(19) Europäisches Patentamt
European Patent Office
Office européen des brevets

(11) EP 2 745 115 B1

(12) # EUROPÄISCHE PATENTSCHRIFT

(45) Veröffentlichungstag und Bekanntmachung des Hinweises auf die Patenterteilung:
**18.05.2016 Patentblatt 2016/20**

(21) Anmeldenummer: **12759382.0**

(22) Anmeldetag: **18.08.2012**

(51) Int Cl.:
***G01N 33/543*** *(2006.01)* ***G01N 33/68*** *(2006.01)*

(86) Internationale Anmeldenummer:
**PCT/EP2012/066152**

(87) Internationale Veröffentlichungsnummer:
**WO 2013/026807 (28.02.2013 Gazette 2013/09)**

(54) **NEUES VERFAHREN ZUR DIAGNOSTIK VON HOCHAFFINEN BINDERN UND MARKERSEQUENZEN**

NOVEL METHOD FOR DIAGNOSIS OF HIGH-AFFINITY BINDERS AND MARKER SEQUENCES

NOUVEAU PROCÉDÉ DE DIAGNOSTIC DE LIANTS DE HAUTE AFFINITÉ, ET SÉQUENCES DE MARQUEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.08.2011 DE 102011081293**
**06.12.2011 DE 102011087841**

(43) Veröffentlichungstag der Anmeldung:
**25.06.2014 Patentblatt 2014/26**

(73) Patentinhaber: **Protagen AG**
**44227 Dortmund (DE)**

(72) Erfinder:
- **MÜLLNER, Stefan**
  **40764 Langenfeld (DE)**
- **SCHULZ-KNAPPE, Peter**
  **30966 Hemmingen (DE)**
- **LÜKING, Angelika**
  **44892 Bochum (DE)**
- **GÖHLER, Heike**
  **44797 Bochum (DE)**
- **SCHWERMANN, Jessica**
  **44229 Dortmund (DE)**

(74) Vertreter: **Simandi, Claus**
**Simandi Patentanwälte**
**Höhenstrasse 26**
**53773 Hennef (DE)**

(56) Entgegenhaltungen:
**EP-A1- 2 441 848**
**WO-A1-2011/038138**
**WO-A2-2010/000874**
**US-A1- 2005 019 843**

- **MCBRIDE JEFFREY D ET AL: "Screening autoantibody profiles in systemic rheumatic disease with a diagnostic protein microarray that uses a filtration-assisted nanodot array luminometric immunoassay (NALIA)", CLINICAL CHEMISTRY, AMERICAN ASSOCIATION FOR CLINICAL CHEMISTRY, WASHINGTON, DC, Bd. 54, Nr. 5, 1. Mai 2008 (2008-05-01), Seiten 883-890, XP002597858, ISSN: 0009-9147, DOI: 10.1373/CLINCHEM.2007.098418 [gefunden am 2008-03-20]**
- **ROBINSON W H ET AL: "Autoantigen microarrays for multiplex characterization of autoantibody responses", NATURE MEDICINE, NATURE PUBLISHING GROUP, NEW YORK, NY, US, Bd. 8, Nr. 3, 1. März 2002 (2002-03-01), Seiten 295-301, XP002521048, ISSN: 1078-8956, DOI: 10.1038/NM0302-295**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 745 115 B1

## Beschreibung

Die vorliegende Erfindung betrifft ein neues Verfahren zur Diagnostik von hochaffinen Bindern, insbesondere Antikörpern und/oder Autoantikörpern, und die Identifizierung, Charakterisierung und Selektion von Markersequenzen und deren diagnostische Verwendung, insbesondere in Form eines Panels.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Es besteht jedoch ein hohes Bedürfnis die Diagnostik von hochaffinen Bindern, insbesondere Antikörpern, Autoantikörpern und Markersequenzen zu verbessern.

MCBRIDE JEFFREY D ET AL: "Screening autoantibody profiles in systemic rheumatic disease with a diagnostic protein microarray that uses a filtration-assisted nanodot array luminometric immunoassay (NALIA)", CLINICAL CHEMISTRY, AMERICAN ASSOCIATION FOR CLINICAL CHEMISTRY, WASHINGTON, DC, vol. 54, no. 5, 1 May 2008, Seite 883-890, WO 2011/038138 A1 und US 2005/019843 A1 betreffen Verfahren zur Identifizierung und/oder Charakterisierung und/oder Selektion von Markersequenzen, wobei n-platzierte Markersequenzen auf einen festen Träger mit einer Probe versetzt werden und Wechselwirkungen mittels Signale nachgewiesen werden, jedoch werden keine hochsensitiven Signalintensitäten erreicht.

Proteinbiochips der Anmelderin sind bereits beschrieben und erlauben die Diagnose von Krankheiten, insbesondere Autoimmunerkrankungen, anhand der Identifizierung von Markersequenzen.

WO2010/000874 der Anmelderin beschreibt z.B. die Diagnose von Prostatakarzinom und Prostataentzündungen mittels eines Proteinbiochip und stellt bestimmte diagnostische Markersequenzen zur Verfügung.

Hierbei konnten erstmals mittels Proteinbiochips diese Markersequenzen für die jeweiligen Indikationen sensitiv identifiziert werden, jedoch werden keine hochsensitiven Signalintensitäten erreicht.

Proteinbiochips erlauben die vorteilhafte Zuweisung einzelner hochdichter Loci (Spots) zu den jeweiligen Markersequenzen und Signalen.

Beispielsweise kann ein Proteinbiochip mit einem hochaffinen Binder aus einer Probe versetzt werden und die Wechselwirkung zwischen einem hochaffinen Binder und einer Markersequenz in einer Gesamtheit verschiedener Markersequenzen nachgewiesen werden. Dies ist ebenfalls mit einem Array möglich, wobei auf einzelnen Spots (Loci) Markersequenzen angeordnet sind.

Auf diese Weise können ebenfalls diagnostische Fragestellungen bearbeitet werden, je nach Auswahl der Markersequenzen und der eingesetzten hochaffinen Binder. Insbesondere sind Diagnoseverfahren von Interesse, wobei ein Proband oder Patient eine Probe enthaltend hochaffine Binder bereitstellt und die Probe auf einen Array mit ausgewählten Markersequenzen zur Diagnose einer Fragestellung oder eines Ereignisses (z.B. Zustand, Krankheit, etc.) gegeben wird.

Es besteht jedoch ein hohes Bedürfnis solche Verfahren zu vereinfachen und zu minimalisieren, insbesondere zu miniaturisieren.

Zumeist können eine Mehrzahl von Markersequenzen für eine bestimmte diagnostische Fragestellung herangezogen werden, und so genannte "Markerpanel" dienen zur besseren Bewältigung diagnostischer Fragestellungen oder Ereignisse. Beispielsweise werden im Stand der Technik literaturbekannte Markersequenzen miteinander kombiniert, so dass eine erhöhte Aussagekraft erzielt werden kann.

Die Aufgabe der vorliegenden Erfindung ist daher die Bereitstellung von verbesserten Diagnoseverfahren und deren diagnostische Verwendung, so dass geeignete Panel von Markersequenzen bereitgestellt werden können.

Eine weitere Aufgabe betrifft die Identifizierung und/oder Charakterisierung und/oder Selektion von Markersequenzen, so dass geeignete Panel von Markersequenzen bereitgestellt werden können.

Die Aufgabe wird gelöst durch ein Verfahren zur Identifizierung und/oder Charakterisierung und/oder Selektion von Markersequenzen mit den folgenden Schritten:

a.) n platzierte Markersequenzen werden auf einem festen Träger mit einer Probe enthaltend hochaffine Binder versetzt und eine Wechselwirkung wird mittels Signalen nachgewiesen,

b.) mindestens n-1 platzierte Markersequenzen mit Signalintensität aus a.) werden auf einem Träger mit einer gleichen Probe enthaltend hochaffine Binder versetzt und eine Wechselwirkung wird mittels Signalen nachgewiesen,

c.) optional wird Schritt b.) mit mindestens n-k Markersequenzen wiederholt, wobei $k \geq 1$ gilt,

d.) n-k ausgewählte Markersequenzen werden auf einem Träger gemeinsam auf einem Locus aufgebracht und mit einer gleichen Probe versetzt und eine Wechselwirkung wird mittels einem Single-Signal nachgewiesen.

Das vorstehende Verfahren wird nachstehend "erfindungsgemäßes Verfahren" genannt.

Die Anzahl von Markersequenzen, die in dem erfindungsgemäßen Verfahren eingesetzt wird, wird mit "n" bezeichnet. Theoretisch können beliebig viele Markersequenzen eingesetzt werden. Die Anzahl ist nur durch die praktische Realisierbarkeit (z.B. Dimension des festen Trägers, verwendete cDNA Bank) begrenzt. Beispielsweise können 50.000 oder 100.000 oder mehr Markersequenzen in Schritt a.) des erfindungsgemäßen Verfahrens eingesetzt werden. Bevorzugt ist die Verwendung von 50.000, 40.000, 30.000, 20.000, 10.000, 5.000, 1.000 oder weniger Markersequenzen in Schritt a.) des erfindungsgemäßen Verfahrens.

Bei gegebenem n von 5.000 ist k bevorzugt ein Wert von 1 bis 1.000, insbesondere 10 bis 500. Beispielsweise kann eine Signifikanz von 90, 95 oder 99 oder 99,9 % der Signalintensitäten eingestellt werden, so dass hinreichend solche Markersequenzen in Schritt b.) berücksichtigt und selektiert werden. Schritt c.) kann ebenfalls mehrfach wiederholt werden.

n ist eine repräsentative Gesamtheit von Markersequenzen auf einem festen Träger. Nachfolgend wird Schritt b.) des erfindungsgemäßen Verfahrens und gegebenenfalls dessen Wiederholung gemäß Schritt c.) durchgeführt, wodurch Markersequenzen mit hinreichender Signalintensität und hinreichender Wechselwirkung mit den hochaffinen Bindern selektiert werden. Dabei bleiben in den weiteren Verfahrensschritten, die Marker unberücksichtigt, die keine hinreichende Signalintensität und keine hinreichende Wechselwirkung mit den hochaffinen Bindern aufweisen. Für die Verfahrensschritte b.) und c.) werden deshalb nur n-1 bzw. n-k Markersequenzen eingesetzt, wobei $k \geq 1$ ist und wobei vorzugsweise $k < n$ ist. Auf diese Weise können in einer besonderen Ausführungsform der Erfindung könnnen beispielsweise "z" spezifische Markersequenzen aus einem einzigen Verfahren erhalten werden, wobei n-k = z. IN einer anderen besonderen Ausführungsform des Verfahrens können die spezifischen Markersequenzen "z" durch mehrere unabhängig voneinander durchgeführte Verfahren erhalten werden.

Im Ergebnis wird mittels des erfindungsgemäßen Verfahrens ein Panel von spezifischen Markersequenzen mit hinreichender Sensitivität erhalten.

Besonders vorteilhaft können Panel von Markersequenzen erhalten werden, die besonders vorteilhaft auf einem Locus oder Spot auf einem Array aufgetragen werden, so dass dieses Panel von Markersequenzen ein Single-Signal emittieren kann.

Überraschender Weise können auf diese Weise besonders effektive Panel von Markersequenzen erhalten werden, die ein Single-Signal mit signifikanter Signalintensität gewährleisten. Dies ist besonders vorteilhaft, falls verschiedene Signale mit unterschiedlichen Signalintensitäten auf einen Array vorliegen. Anderseits erlaubt "ein Locus-ein Signal" (Single-Signal) für ein Panel von Markersequenzen eine hinreichende Miniaturisierung und Vereinfachung der Diagnostik, so dass nicht z Signalintensitäten von z Markersequenzen ermittelt werden müssen, sondern lediglich eine Signalintensität des einzellokalisierten Panel von z Markersequenzen. Dies ist ebenfalls vorteilhaft für das Signal-Rausch-Verhältnis im Rahmen der Auswertung, insbesondere mittels algorithmischen Methoden.

Ein Single-Signal kann beispielsweise der Mittelwert oder der Median der gemessenen oder berechneten Signalintensitäten der einzelnen Markersequenzen in dem Panel sein.

Die Bereitstellung von solchen Paneln mit Markersequenzen erlaubt zudem eine sichere Diagnose eines Ereignisses eines Probanden (z.B. Zustand, Krankheit) und / oder Stratifizierung von Patienten mit einer Erkrankung.

"Diagnose" bedeutet die positive Feststellung einer Wechselwirkung zwischen Markersequenzen und hochaffinen Bindern, insbesondere Antikörpern, Autoantikörpern. Ferner kann aufgrund dieser Wechselwirkung auf ein Ereignis geschlossen werden. Bevorzugt ist das Ereignis die Aussage über eine Krankheit oder einen Zustand eines Probanden oder Patienten. Der Begriff der Diagnose umfasst die medizinische Diagnostik und diesbezügliche Untersuchungen, insbesondere die in-vitro Diagnostik und Labordiagnostik.

"Stratifizieren (auch: Stratifikation) oder Therapiesteuerung" bedeutet, dass das erfindungsgemäße Verfahren Entscheidungen zur Behandlung und Therapie des Patienten erlaubt, sei es die Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten. Insbesondere umfasst ist ebenfalls die personalisierte Medizin.

In einer weiteren beschriebenen Ausführungsform umfasst der Begriff "Stratifizierung" insbesondere die Risikostratifizierung mit der Prognose eines "outcome" eines nachteiligen gesundheitlichen Ereignisses.

Es wird unter dem Begriff "Patient" ein beliebiger Proband - Mensch oder Säugetier - verstanden.

Der Begriff "Markersequenzen" im Sinne dieser Erfindung umfasst solche Moleküle, die mittels hochaffiner Binder erkannt werden können. Insbesondere sind daher nicht abschließend solche Sequenzen wie mRNA, si-RNA, microRNA, cDNA, Peptid oder Protein, insbesondere Antigene oder Autoantigene, umfasst. Die Markersequenzen können aus einer Expressionsbibliothek stammen, insbesondere eine mRNA, si-RNA, microRNA, cDNA, Peptid oder Protein-Expressionsbibliothek umfassen. Weiterhin können die Markersequenzen aus jedem biologischem Material gewonnen werden, solche wie nicht abschließend Gewebe, native Quellen, Zellen, Bakterien, Viren, Phagen, Prionen, Pflanzen, Tiere, Menschen, etc. Vorzugsweise werden die Markersequenzen nach gängigen Verfahren gereinigt und ggfs. isoliert.

Diese Markersequenzen binden signifikant mit einem hochaffinen Binder und weisen zu dem hochaffinen Binder eine signifikante Wechselwirkung auf. Beispielsweise können die cDNA oder das jeweils daraus erhältliche Polypeptid oder Protein eine Wechselwirkung mit Substanzen aus der Körperflüssigkeit oder Gewebeauszug eines Patienten mit einer Erkrankung aufweisen (z.B. Antigen (Epitop) / Antikörper (Paratop) Wechselwirkung).

Eine solche Wechselwirkung ist z.B. eine Bindung an mindestens einer Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Im Rahmen dieser Erfindung ist ein hochaffiner Binder ein solcher, der spezifisch an eine "Markersequenz" bindet oder an eine solche "Markersequenz" adressiert ist, bevorzugt solche wie Antikörper oder Autoantikörper. Antikörper binden mit einer oder mehreren Antigen-Bindungsregionen an ein oder mehreren Epitopen der Markersequenz spezifisch nach dem Schlüssel-Schloss Prinzip und bilden auf diese Weise eine signifikante Wechselwirkung im Sinne der Erfindung.

Im Sinne der Erfindung liegt beispielsweise eine spezifische Wechselwirkung vor, wenn die Dissoziationskonstante von hochaffinem Binder und Markersequenz im picomolaren oder nanomolaren Bereich liegt.

Solche hochaffinen Binder, insbesondere (Auto)Antikörper sind erfindungsgemäß vorzugsweise Bestandteil einer Probe eines Patienten/Probanden, beispielsweise einer Probe der Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

Die "Markersequenzen" verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion und/oder ein Aptamer.

Erfindungsgemäß umfassen die Markersequenzen auch Modifikationen einer cDNA-Sequenz oder einer Aminosäuresequenz, wie chemische Modifikationen, insbesondere Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente von bekannten Markersequenzen (z.B. SWISSProt, Datenbanken,etc.) umfasst. Insbesondere solche Teilsequenzen, die eine Identität von 95%, 90 %, insbesondere 80% oder 70 % mit den bekannten Markersequenzen aufweisen.

In einer weiteren Ausführungsform können die jeweiligen Markersequenzen in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Weiterhin ist erfindungswesentlich, dass mindestens ein Spot oder Locus ein Panel von Markersequenzen aufweist, beispielsweise 10 oder mehr, vorzugsweise 5, 6 oder 7, besonders bevorzugt, 2, 3 oder 4 Markersequenzen.

Der Begriff "Anordnung" bedeutet synonym "Array" und sofern dieser "Array" zur Identifizierung von Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Markersequenzen erlauben und die Markersequenzen gespottet werden. Solche hochdichte gespottete Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Offenbarung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren. Ein Proteinbiochip im Sinne dieser Erfindung ist die systematische Anordnung von Proteinen auf einem festen Träger.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60 (5) : 523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden. Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einem festen Träger fixiert, gespottet oder immobilisiert.

Daher ist ebenfalls eine Anordnung beschrieben, wobei die Markersequenzen als Clone vorliegen.

Zusätzlich können die Markersequenzen in der jeweiligen Form in Form eines Fusionsproteins vorliegen, welches beispielsweise mindestens ein Affinitätsepitop oder "Tag" enthält. Der Tag kann ein solcher sein, wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix aufweist.

In einer weiteren Ausführungsform betrifft die Offenbarung einen Assay oder Proteinbiochip zum Identifizieren und Charakterisieren eines hochaffinen Binders für die jeweiligen Markersequenzen, dadurch gekennzeichnet, dass eine Anordnung oder Assay mit a.) mindestens einem hochaffinen Binder in Kontakt gebracht wird und b.) eine Wechselwirkung mittels einem Signal nachgewiesen wird, wobei mindestens ein Locus (Spot) einen Panel von Markersequenzen aufweist, erhältlich nach dem erfindungsgemäßen Verfahren.

In einer bevorzugten Variante der Erfindung ist der hochaffine Binder mindestens ein Autoantikörper, welcher mindestens ein Panel von Markersequenzen erhältlich nach dem erfindungsgemäßen Verfahren erkennt, wobei aufgrund des eingesetzten Autoantikörpers in der Probe des erfindungsgemäßen Verfahrens n-k Markersequenzen in einem Panel bereitgestellt werden. Insbesondere werden vorzugsweise die Markersequenzen aus Patienten oder Probanden erhalten, die eine Krankheit aufweisen, die maßgeblich mit der Prominenz solcher Autoantikörper korrelieren. Z.B. sind Autoantikörper besonders prominent bei Patienten mit Autoimmunerkrankungen und entsprechende Autoantigene können mittels dem erfindungsgemäßen Verfahren identifiziert, charakterisiert und selektiert werden und insbesondere in einem Panel von Markersequenzen bereit gestellt werden.

Von daher können Probanden und Patienten vorteilhaft auf die entsprechende Erkrankung diagnostiziert oder stratifiziert werden. Weiterhin kann eine personalisierte Medizin erfolgen, indem ein patientenindividuelles Panel von Markersequenzen hergestellt wird, indem beispielsweise patienteneigenes Gewebe (biologisches Material) als Ausgangsmaterial verwendet wird.

Daher betrifft die Offenbarung ebenfalls Panel von n-k Markersequenzen erhältlich nach dem erfindungsgemäßen Verfahren auf einem festen Träger, wobei die n-k Markersequenzen mit einem Single-Signal korrelieren und auf einem Locus präsentiert sind, und die Markersequenzen spezifische Autoantigene sind, die spezifisch an die komplementären Autoantikörper einer Probe enthaltend Autoantikörper binden.

In einer weiteren Ausführungsform betrifft die Offenbarung die Diagnose oder Stratifizierung von Erkrankungen mittels einem Panel von Markersequenzen, wobei die Markersequenzen beispielsweise Autoantigene sind, auf einem Locus (Spot) eines festen Trägers, wobei eine Probe enthaltend Autoantikörper in Kontakt gebracht und eine Wechselwirkung mittels einem Single-Signal nachgewiesen wird.

Ein Locus (Spot) ist vorzugsweise auf einem festen Träger angeordnet. Der Locus kann einem Well einer Mikrotiterplatte oder einem Gitterpunkt im Luminex Assay entsprechen. Der Locus ist das Testfeld, in dem sich das Panel aus Markersequenzen befindet und von seinen Dimensionen, seiner Topographie und dem verwendeten Material so ausgestaltet das die Markersequenzen aufgebracht, mit dem hochaffinen Binder in Kontakt gebracht und ein Single-Signal erzeugt und gemessen werden kann.

Gegenstand der Offenbarung ist auch ein Singleplex Assay umfassend ein Panel von 2 oder mehr verschiedenen Markersequenzen mit hinreichender Sensitivität auf einem Locus oder Spot, wobei die verschiedenen Markersequenzen an einen oder mehrere hochaffine Binder binden können und dadurch ein Single-Signal zum Nachweis des oder der hochaffinen Binder(s) erhalten wird.

Eine Ausführungsform der Offenbarung betrifft einen Singleplex Assay wobei 10 oder 50 oder 100 oder mehr, vorzugsweise 6, 7 oder 8, besonders bevorzugt 3, 4 oder 5 verschiedene Markersequenzen kombiniert werden und dadurch das Panel verschiedener Markersequenzen bilden.

Eine weitere Ausführungsform der Offenbarung betrifft einen Singleplex Assay, wobei die verschiedenen Markersequenzen durch das erfindungsgemäße Verfahren erhalten werden. Vorzugsweise betrifft die Offenbarung eine Ausführungsform bei der für den Singleplex Assay Markersequenzen aus Antigenen, Teilen von Antigenen, Haptenen oder Proteinen ausgewählt werden.

Gegenstand der Offenbarung ist auch die Verwendung eines Singleplex Assays zur Früherkennung und/oder Diagnose und/oder Stratifizierung einer Erkrankung.

Gegenstand der Offenbarung ist auch ein Kit zur Früherkennung und/oder Diagnose und/oder Stratifizierung von Erkrankungen enthaltend ein Panel von Markersequenzen die nach dem erfindungsgemäßen Verfahren identifiziert wurden auf einem Träger oder einem Singleplex Assay und weiteren üblichen Hilfsmitteln (Nachweisreagenzien, etc.). In einer weiteren Ausführungsform werden Antigene, insbesondere Autoantigene als Markersequenzen eingesetzt. Eine besondere Ausführungsform betrifft die Anwendung des Kits bei Autoimmunerkrankungen oder Verdacht auf Autoimmunerkrankungen (Früherkennung).

Erkrankungen umfassen beliebige Erkrankungen des Menschen, vorzugsweise solche wie Krebs, Autoimmunerkrankungen, insbesondere rheumatoide Arthritis, Multiple Sklerose u.a., vorzugsweise solche Erkrankungen, die charakteristische Autoantikörper bzw. Muster von Autoantikörpern, vorzugsweise im Plasma oder Serum aufweisen.

Nachdem der hochaffine Binder das Panel von Markersequenzen auf einem Locus (Spot) kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Wechselwirkungen (z.B. Protein an Markersequenz, wie Antigen/Antikörper) oder entsprechende "Mittel zum Nachweis eines Bindungserfolges" bzw. "Mittel zum Nachweis einer Wechselwirkung mittels Signalen" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, kolloidale Gold- oder Latex-Partikel), oder mit Reporter-Enzymen wie alkalischer Phosphatase, Meerrettichperoxidase, usw. und den entsprechenden kolorimetrischen, fluoreszenten oder chemolumineszenten Substraten. Eine Auslesung erfolgt z.B. mittels eines Microarray-Laserscanners, einer CCD-Kamera oder visuell.

Weiterhin kann die Bestimmung von Signalen zum Nachweis einer Wechselwirkung mit Hilfe von radioaktiven oder fluoreszenzmarkierten Antikörpern erfolgen, insbesondere mittels bioanalytischer Verfahren, wie Western blot (1D und 2D), Immunhistochemie, Antikörperarrays, Luminex®, ELISA, Immunfluoreszenz, Radioimmunoassays. massenspektrometrische Verfahren, wie MRM (Multi reaction monitoring) oder AQUA (absolute Quantification).

Figuren 1 bis 4 zeigen die Mittelwert MFIs von Multiplex Assay (mehrere Markersequenzen liefern mehrere einzelne Signale) und Singleplex Assays im Vergleich, wobei 10 verschiedene Antigene (Proteine 1 bis 10) und die Seren RA00029 (Figur 1), RA0037 (Figur 2), RA00046 (Figur 3) und PRO244 (Figur 4) verwendet wurden. Es wurden die Einzelwerte für Proteine 1 bis 10 im Multiplex Assay gemessen und daraus die Mittelwerte für den Multiplex Assay berechnet und mit dem gemessenen Wert (Single-Signal) für den Singleplex Assay verglichen.

Figur 5 zeigt den Vergleich der Median MFIs von Multiplex und Singleplex Assay, wobei 10 verschiedene Antigene (Proteine 1 bis 10) verwendet wurden. Als Kopplungskontrolle wurden 10 $\mu$l/ml (Median der gezählten Beads). Set 1 (Multiplex Assay) mit 10 Proteinen an unterschiedlichen Beadregionen im Vergleich zu Set 2 (Singleplex Assay) mit 10 Proteinen an der gleichen Beadregion.

Figur 6 zeigt den Vergleich der Mittelwert MFIs von Multiplex und Singleplex Assay, wobei 10 verschiedene Antigene (Proteine) verwendet wurden. Als Kopplungskontrolle wurden 10 $\mu$l/ml (Mittelwert der gezählten Beads). Set 1 (Multiplex Assay) mit 10 Proteinen an unterschiedlichen Beadregionen im Vergleich zu Set 2 (Singleplex Assay) mit 10 Proteinen an der gleichen Beadregion.

Figur 7 zeigt die Spots auf einem Mikroarray, auf dem Proteine und Proteingemische (Markersequenzen) immobilisiert sind, die als Antigene fungieren und die mit Serum RA00037 inkubiert wurden.

Figur 8 zeigt die Spots auf einem Mikroarray, auf dem Proteine und Proteingemische (Markersequenzen) immobilisiert sind, die als Antigene fungieren und die mit Serum RA00033 inkubiert wurden.

Figur 9 zeigt den errechneten Mittelwert im Vergleich zum Messwert der Signalintensitäten bei Verwendung von Serum RA00037 mit verschiedenen Proteinen und Proteingemischen als Markersequenz bzw. Panel von Markersequenzen.

Figur 10 zeigt den errechneten Mittelwert im Vergleich zum Messwert der Signalintensitäten bei Verwendung von Serum RA00033 mit verschiedenen Proteinen und Proteingemischen als Markersequenz bzw. Panel von Markersequenzen.

Figur 11 zeigt Tabelle 1. Angegeben sind die Signalintensitäten der verwendeten Seren (RA0026 bis RA0046) bei den einzelnen verwendeten Markersequenzen P1 bis P9 und P0.

Nachfolgend wird die Erfindung durch Beispiele erläutert. Die Erfindung ist jedoch nicht auf die Beispiele beschränkt sondern grundsätzlich universell für verschiedenste Markersequenzen, hochaffine Binder, Typen von Assays und Nachweisverfahren anwendbar.

In den Beispielen werden ELISA und SUPA Luminex Assays verwendet, außerdem Antigene (Proteine) als Markersequenzen, sowie Seren, die Antigene und Autoantigene als hochaffine Binder enthalten.

Assays auf Basis der Luminex Technologie erlauben die simultane quantitative Bestimmung von bis zu 100 Parametern in einer einzelnen Probe, wobei im Stand der Technik Multiplex Assays bekannt sind. Vorteile des Multiplex Formats sind hohe Sensitivität und Spezifität, exakte Quantifizierung und Automatisierbarkeit.

Eine bevorzugte Ausführungsform der Erfindung betrifft Singleplex Assays (Single-Signal Assays). In dem erfindungsgemäßen Singleplex Assay (Single-Signal Assay) wird im Gegensatz zum Multiplex Assay nur ein Parameter bestimmt. Bei dem erfindungsgemäßen Singleplex Assay wird nicht eine ausgewählte Markersequenz sondern ein Panel von Markersequenzen verwendet das durch Kombination von mehreren Markersequenzen zu einem einzigen Marker (Panel von Markern, Markerkombination) erhalten wird. Ein Vorteil bei der Verwendung eines Panels von Markersequenzen in einem Singleplex Assay ist die Additive Signalverstärkung durch die Kombination der Markersequenzen in einem Panel. Gemessen wird bei dem Singleplex Assay dann vorzugsweise der Mittelwert der Intensität der kombinierten Markersequenzen. Markersequenzen werden nachfolgend auch einfach als Marker bezeichnet.

<u>Beispiel 1:</u> Additive Signalverstärkung durch Markerkombination in einem Panel von Markerproteinen.

Theoretische Signalintensität, Vorüberlegung

Bei einem Singleplex Assay mit einem Panel aus Markersequenzen aus Protein 1 und Protein 2 (Zweifachkombination), bei dem das Protein 1 eine Signalintensität von 10.000 und Protein 2 eine Signalintensität von 30.000 hat, beträgt der berechnete Mittelwert der Intensität MFI der Signale aus Protein 1 und Protein 2 20.000.

Bei einem Singleplex Assay mit einer Fünffachkombination aus Protein 1, Protein 2, Protein 3, Protein 4, Protein 5 in einem Panel entspricht die berechnete Signalintensität des Panels aus Markersequenzen dem Mittelwert der Einzelsignale der 5 Proteine.

Versuchsdurchführung

Die verwendeten Beads sind mit den Proteinen die als Antigen fungieren beschichtet. Diese beschichteten Beads werden mit unterschiedlichen Seren inkubiert, so dass die im Serum vorhandenen Antikörper an die Antigene binden können. Es geht dabei darum, die Signalintensitäten einer Multiplex Messung mit der Signalintensität der erfindungsgemäßen Singleplex Messung zu vergleichen.

Bei dem Mulitplex Assay werden 10 unterschiedliche Beadregionen mit 10 unterschiedlichen Proteinen beschichtet, wobei 100 Beads/Beadregion - d.h. insgesamt 1000 Beads - eingesetzt werden. Es werden dann 45 Seren (je 2 Replikate) in drei verschiedenen Settings getestet: Als 10 Plex mit den Proteinen 1-10 und als 5 Plex, einmal mit den Proteinen 1-5 und einmal mit den Proteinen 6-10. Bei der Datenauswertung wird der Median MFI (Median Fluorescent Intensity) oder Mittelwert MFI (Mean Fluorescent Intensity) aller Proteine bestimmt.

Bei dem Singleplex Assay werden 10 Proteine in der gleichen Beadregion getestet, wobei 1.000 Beads sich in einer Beadregion befinden. Getestet werden 45 Seren (je zwei Replikate) in 3 Settings: als Singleplex mit den Proteinen 1-10, als Singleplex mit den Proteinen 1-5 und als Singleplex mit den Proteinen 6-10. Bei der Datenauswertung wird der Median MFI oder Mittelwert MFI der 1000 Beads (Mix) berücksichtigt.

Das Prinzip der Messung und Auswertung beruht auf der Luminex Technologie. Die Beads sind mit zwei Fluoreszenzfarbstoffen (rot und infrarot) eingefärbt, die in unterschiedlichen Bereichen des optischen Spektrums emittieren. Die Kombination dieser beiden Farbstoffe in jeweils zehn verschiedenen Konzentrationsstufen führt zu einhundert spektral unterscheidbaren Schattierungen von rot und infrarot. Jede der daraus resultierenden Fluoreszenzintensitäten definiert eine Population von Beadregionen. Die Fluoreszenzkodierung der Beadregionen sind die Basis für die Erkennung durch das Analysegerät und die exakte Zuordnung. Die eindeutige Zuordnung der einzelnen Beadregionen ist die Grundlage für die Multiplexe Analyse, wobei jede Beadregion einen Einzeltest repräsentiert (Fluoreszenzkodierung der Beadregionen).Im Singleplex wird nur eine Beadregion verwendet, so dass ein Einzeltest durchgeführt wird und eine Zuordnung als auch Markierung mit dem roten und infraroten Fluoreszenzfarbstoff im Gegensatz zum Multiplex Assay nicht erforderlich ist.

Die Beads werden mit den zu untersuchenden Proteinen 1 bis 10 beschichtet, die als Antigen wirken. Die Beads werden dann mit den zu untersuchenden Seren inkubiert. Je besser die in einem Serum vorhandenen Antikörper an das oder die auf dem Bead immobilisierten Antigene (Markerproteine) binden, desto höher ist das gemessene Fluoreszenzsignal. Der spezifische Nachweis der Bindung des Antikörpers an die Beads erfolgt über ein Detektionsmolekül (Konjugat). Dieses Konjugat hat eine hohe spezifische Affinität zum gebundenen Antikörper aus dem Serum und ist mit einem Fluoreszenzfarbstoff (z.B. Phycoerythrin) gekoppelt, der im Wellenlängenbereich des grünen Lichts emittiert. Dieser spektrale Bereich unterschiedet sich von denen der internen Farbstoffe, so dass die Klassifizierung der Beads und die Quantifizierung der Antikörper im Multiplex Assay neben einander durchgeführt werden können.

Auswertung der Ergebnisse:

Die allgemeine Standardauswertung für die Messung von Luminexbeads ist der Median MFI (Median Fluorescent Intensity). Dabei werden systematisch einzelne hohe bzw. niedrige Signale vernachlässigt.

Der Median teilt die Gesamtmenge (Anzahl) der gemessenen Intensitäten in zwei Hälften. Der Median ist somit der Intensitätswert, der in der Mitte aller gemessenen Intensitäten liegt. Er gibt die mittlere Intensität der gemessenen Intensitäten an, die "typische" Intensität für ein bestimmtes Protein, hier einen bestimmten Antikörper. Bei der Angabe des Medians, wird nur der mittlere Wert berücksichtigt, während einzelne hohe und niedrige Intensitätssignale, die rechts und links vom Median liegen unberücksichtigt bleiben.

Der Mittelwert "M" (arithmetisches Mittel) berechnet sich wie folgt:

$$M = 1/n \Sigma Xi = x1+x2+ \ldots .+xn / n$$

Damit ergibt sich für die Multiplex Messung eines Proteins 1 an Bead 1 (Signalstärke 300), Protein 2 an Bead 2 (Signalstärke 600) und Protein 3 an Bead 3 (Signalstärke 30.000) ein errechneter Median von 600 MFI (Median Fluorescent Intensity) und ein errechneter Mittelwert von 10.3000 MFI (Mean Fluorescent Intensity).

Für eine Singleplex Messung von Protein 1 (Signalstärke 300), Protein 2 (Signalstärke 600) und Protein 3 (Signalstärke 30.000) an einem Bead gemessener Median von 600 MFI und ein gemessener Mittelwert von 10.300 MFI.

Die Werte von Median MFI und Mittelwert MFI in Multiplex und Singleplex Assay sind folglich identisch.

In der erfindungsgemäßen Singleplex Messung sollen einige hohe Signale im Gegensatz zur herkömmlichen Multiplex Messung nicht vernachlässigt werden. Daher wird bei der erfindungsgemäßen Singleplex Messung vorzugsweise der Mittelwert bestimmt, und nicht der Median.

In Figur 1 werden die MFI Mittelwerte der einzelnen Proteine 1 bis 10 des Multiplex Assays dargestellt. Aus diesen Werten wurden der Mittelwert des Multiplex Assays für Proteine 1-10, 1-5 und 6-10 berechnet. Diese berechneten Mittelwerte des Multiplex Assays wurden mit den aus den gemessenen Werten des Singleplex Assays für die Proteine 1-10, 1-5 und 6-10 verglichen.

Es wurden die Seren RA-00037 (positiv), RA-00029 (positiv), RA-00046(positiv) und PRO-244 (negativ) untersucht.

Bei dem Serum RA-00029 zeigt sich, dass die berechneten Mittelwerte des Multiplex Assays mit den gemessenen Mittelwerten des Singleplex Assays weitgehend übereinstimmen (d.h. vergleichbar sind) und zwar sowohl für die Proteine 1-10, als auch für die Proteine 1-5 und 6-10.

Für die Seren RA-00037 (Figur 2), RA-00046 (Figur 3) und PRO-244 (Figur 4) werden ähnliche Effekte gefunden - die aus den gemessenen Intensitäten berechneten Mittelwerte der Multiplex Assays sind mit den gemessenen Intensitäten des Singleplex Assays (Auswertung nach Mittelwerten) vergleichbar.

Diese Experimente haben gezeigt, dass der Mittelwert MFI des Multiplex Assays (Proteine auf unterschiedlichen Beadregionen) als 5 Plex als auch als 10 Plex vergleichbar ist mit dem Mittelwert des jeweiligen Singleplex Assays (Proteinpanel auf einer Beadregion).

<u>Beispiel 2</u>

Vergleich von Multiplex Assay und Singleplex Assay im Bezug auf die Median Intensitäten und die Mittelwert Intensitäten

Versuchsaufbau und Durchführung wie in Beispiel 1 mit Protein 1 an Bead 1, Protein2 an Bead 2 und Protein 3 an Bead 3 beim Muliplex Assay. Aus den gemessenen Intensitäten der Proteine 1 bis 3 wurde einmal der Median MFI und einmal der Mittelwert MFI für den Multiplex Assay bestimmt.

Diese wurden mit den gemessenen Intensitäten des Median MFI und des Mittelwertes MFI des Singleplex Assays verglichen. Beim Singleplex Assay waren die Proteine 1, 2 und 3 an einem Bead (Bead 4) immobilisiert.

Figur 5 zeigt den Vergleich des Medians von Multiplex und Singleplex Assay der einzelnen Proteine 1 bis 10. Im Set 1 sind die Proteine 1-10, 1-5 und 6-10 an unterschiedliche Beadregionen gekoppelt und in Set 2 sind die Proteine 1 bis 10, 1-5 und 6-10 separat an die gleiche Beadregion gekoppelt. Die Werte für den Median MFI von Multiplex und Singleplex Assay sind für die Proteine 1 bis, 1-5 und 6-10 nahezu gleich. Als Kopplungskontrolle wurden 10 µg/ml (Median der gezählten Beads) eingesetzt.

Figur 6 zeigt den Vergleich des Mittelwerts von Multiplex und Singleplex Assay der einzelnen Proteine 1 bis 10. Im Set 1 sind die Proteine 1-10, 1-5 und 6-10 an unterschiedliche Beadregionen gekoppelt und in Set 2 sind die Proteine 1 bis 10, 1-5 und 6-10 separat an die gleiche Beadregion gekoppelt. Die Werte sowohl für den Mittelwert MFI von Multiplex und Singleplex Assay sind nahezu gleich. Als Kopplungskontrolle wurden 10 μg/ml (Mittelwert der gezählten Beads) eingesetzt.

Beispiel 3: Auswertung SUPA Mikroarray

Verglichen wurden die Signalintensitäten von Mulitplex und Singleplex Assay. Untersucht wurde, wie sich die Signalintensität bei einer Reduktion von Markersequenzen durch die Kombination von mehreren Markersequenzen zu einem Markerpanel verändert.

Ausgangspunkt ist folgende Überlegung: Die Summe der Signalintensitäten 15.000 und 5.000 ergibt eine Signalintensität von 20.000. Der Mittelwert der Signalintensität aus 15.000 und 5.000 ist eine Signalintensität von nur 10.000.

In Mikroarray Experimenten wurden die Antigen/Antikörper Interaktionen von 10 Proteinen und 20 Seren auf Protein Mikroarrays untersucht (siehe Tabelle 1, Abb.11). Die untersuchten Antigene (Proteine) sind mit P1 bis P0 bezeichnet. Es wurden die Seren RA00026 - RA00033, RA00035 - RA00046 auf die Aktivität von in diesem Seren enthaltenen Autoantikörpern untersucht.

Insgesamt wurden nur geringe Signalintensitäten für Protein/Autoantikörper Interaktionen in den einzelnen Seren gefunden, wobei sich die einzelnen Seren hinsichtlich ihrer Aktivität in Bezug auf einzelne Proteine und auch die Signalintensität zum Teil deutlich unterschieden. Es wurden zwei sehr hohe Signalintensitäten von 15.643 (bei Protein 9) und 16.034 (bei Protein 0) mit dem Serum RA00037 gefunden. Serum RA000333 zeigte insgesamt eine mittlere Aktivität - eine Signalintensität von 1.367 bei Protein 1, 1.573 bei Protein 3, 5.010 bei Protein 8 und 2.631 bei Protein 9. Als hohe Signalintensitäten werden solche mit einem MFI (Median Fluorescent Intensity) von größer 10.000 bezeichnet. Als mittlere Signalintensität solche mit einem MFI (Median Fluorescent Intensity) von 1.000 bis 10.000 und als gering solche mit einem MFI von kleiner 1.000.

Die Seren RA00037 und RA00033 wurden im Bezug auf ihre Reaktivität auf verschiedene Proteinkombinationen weiter charakterisiert.

Tabelle 2 gibt einen Überblick über die verwendeten Proteinkombinationen und die verwendete Bezeichnungssystematik.

Tabelle 2

| | Getestete Anzahl | Bezeichnungssystematik | Erläuterung |
|---|---|---|---|
| 1 Protein | 10 | P6 | Protein 6 |
| Kombination von 2 Proteinen | 45 | P67 | Protein 6 und 7 |
| Kombination von 3 Proteinen | 20 | P678 | Protein 6, 7 und 8 |
| Kombination von 4 Proteinen | 8 | P6789 | Protein 6 7, 8 und 9 |
| Kombination von 5 | 2 | P67890 | Protein 6, 7, 8, 9 und 0 |
| Kombination von 10 Proteinen | 1 | P1234567890 | 1, 2, 3, 4, 5, 6, 7, 8, 9 und 0 |

Serum RA00037 und Serum RA00033 wurden mit den verschiedenen Proteinen in den in Tabelle 2 genannten Kombinationen getestet. Es wurden 16 Replikate pro Protein bzw. Proteingemisch getestet. Dann wurde statistisch der Schwellenwert bestimmt, bei dem sich der Background von dem Signal unterscheidet und alle Signale unterhalb des Schwellenwertes (detection call) wurden auf Null gesetzt. Der Detection Call Algorithmus hilft zu entscheiden, ob ein Spot auf einem Microarray leuchtet oder nicht. Dazu wird die Verteilung des lokalen Hintergrundes auf dem Microarray berechnet.

Von dieser Verteilung werden jeweils 5% am unteren Ende (niedrigste Werte) und 5 % am oberen Ende (höchste Werte) entfernt. Dann werden die restlichen Daten zur Basis 10 logarithmiert.

Der Schwellwert ergibt sich aus dem Mittelwert der logarithmierten Daten plus zweimal der Standardabweichung der logarithmierten Daten.

Alle Spots deren Intensitäten unter diesem Wert liegen werden auf Null gesetzt.

Mit dem Serum RA00037 wird das stärkste Signal mit einer Signalstärke von 16034 bei P0 gemessen (Figur 7). Der Background liegt bei einer Signalstärke von 1134 und der dynamische Bereich somit bei 1,15 - d.h. zwischen log 10(1134) und log 10(16034). Insgesamt wurden 35 unterschiedliche Interaktionen bei RA00037 und der verwendeten Proteinkombination detektiert.

Mit dem Serum RA00033 wird das stärkste Signal mit einer Signalstärke von 5010 bei P8 gemessen (Figur 8). Der Background liegt bei einer Signalstärke von 1522 und der dynamische Bereich somit bei 0,52 - d.h. zwischen log10(1522) und log10(5010). Insgesamt wurden 38 unterschiedliche Interaktionen bei RA00033 und der verwendeten Proteinkombination detektiert.

Eine detaillierte Analyse der erzeugten Signalintensitäten der Proteine und Proteinkombinationen durch Interaktion mit Autoantikörpern in Serum RA00037 und Serum RA00033 ist in Tabelle 3 gezeigt.

Tabelle 3

| Bezeichnungssystematik | Serum RA00037 Signalintensität MFI | Bezeichnungssystematik | Serum RA00033 Signalintensität MFI |
|---|---|---|---|
| P6 | 0 | P6 | 0 |
| P7 | 0 | P7 | 0 |
| P8 | 0 | P8 | 5010 |
| P9 | 15643 | P9 | 2631 |
| P0 | 16034 | P0 | 0 |
| P60 | 5236 | P68 | 2218 |
| P70 | 4059 | P78 | 2121 |
| P80 | 5373 | P89 | 3196 |
| P90 | 9856 | P80 | 1653 |
| P670 | 2210 | P678 | 0 |
| P680 | 2490 | P689 | 1809 |
| P690 | 4344 | P680 | 900 |
| P780 | 2014 | P789 | 2331 |
| P790 | 5292 | P780 | 0 |
| P890 | 4783 | P890 | 926 |
| P6790 | 3118 | P6789 | 976 |
| P6890 | 2455 | P6890 | 0 |
| P7890 | 2893 | P7890 | 978 |
| P67890 | 2240 | P67890 | 0 |
| P1234567890 | 0 | P1234567890 | 0 |

Figur 9 zeigt für Serum RA00037 die berechneten und gemessenen MFIs für die einzelnen Proteine und Proteinkombinationen im Vergleich. Figur 10 zeigt dies analog für RA00033.

Es zeigt sich, dass bei der Kombination von vielen Proteinen, die Signalstärke abnimmt und die gemessenen Signalintensitäten unter den berechneten Signalintensitäten liegen. Für die hier untersuchten Seren RA00037 und RA00033 sind bei der Kombination von 10 Proteinen in einer Beadregion keine Signale mehr detektierbar. Bei einer Kombination von 5 Proteinen sind Signale detektierbar, aber die detektierte Signalintensität liegt unter der berechneten. Bei den verwendeten Seren und Markerpaneln sind Panel von 2 bis 3 Proteinen optimal. Insbesondere sind Proteine zur Verwendung in einem Markerpanel geeignet die eine starke Interaktion mit Autoantikörpern zeigen.

Beispiel 4 Singleplex Assay als SUPA-ELISA (Multimarker ELISA)

Die Vorteile des ELISA sind die niedrigen Kosten, die einfache Anwendung (ELISA Anwendungen sind bekannt und standardisiert) und die vergleichsweise schnelle Durchführung.

ELISAs (Enzym-linked-immunoabsorbent-assays) sind seit langem bekannt (z.B. "Immunoassays of endogenous plasma insulin in man", Rosalyn S. Yalow and Solomon A. Berson, From the Radioisotope Service, Veterans Administration Hospital, New York, N. Y., Submitted for publication March 7, 1960; accepted March 22, 1960; J. Clin.

Invest. 39: 1157-75, doi:10.1172/JCI104130. PMC 441860. PMID 13846364; "Enzym-linked immunosorbent assay (ELISA) Quantitative assay of immunoglobulin G" in Immunochemistry, Pergamon Press 1971, Vol. 8 pp. 871-874).

Hier wird der indirekte ELISA verwendet, z.B. beschrieben in (http://en.wikibooks.org/wiki/Structural_Biochemistry/Protein/ Enzyme-Linked_Immuniabsorbent_Assay_%28ELISA%29). Dabei wird eine Vertiefung einer ELISA Platte (well) mit einem Protein beschichtet, das als Antigen fungiert. Daran kann ein spezifischer Antikörper binden. Die Bindung wird durch einen zweiten - enzymgebundenen Antikörper - der ein nachweisbares Substrat umsetzt, nachgewiesen.

Bei einem erfindungsgemäßen Singleplex ELISA werden in einem well einer ELISA Platte 10 Proteine als Antigen eingesetzt und das Signal des Panels aus Antigenen bestimmt.

Die Proteine, die als Antigene verwendet werden, werden über Nacht immobilisiert (dreimal pro Probe). Dann wird gewaschen, mit Candor Puffer für 2 Stunden geblockt, anschließend gewaschen und eine Stunde mit dem zu untersuchenden Serum inkubiert. Daran schließt sich ein Waschschritt und die anschließende einstündige Inkubation mit humanem AK HRP Konjugat. Danach wird abermals gewaschen und mit Substrat 15 min inkubiert. Die Reaktion wird nach 15 min gestoppt und das Ergebnis bei 450 nm (Readout Tecan Safire) ausgelesen.

Es werden drei verschiedene Seren RA00029, RA00037 und RA00045 getestet.

Die Seren liefern die gleichen Ergebnisse wie im Luminex Assay (Beispiel 2 und 3). Es ist notwendig, die Versuchbedingungen für den ELISA für die zu testenden Markerproteine und die zu analysierenden Seren zu optimieren, z.B. im Hinblick auf die verwendeten Puffer und die Beschichtung der ELISA Platten. Der Singleplex Assay ist universell einsetzbar, wobei für den jeweiligen Assaytyp und das verwendete Nachweissystem die Bedingungen optimiert werden müssen, um ein signifikantes Single-Signal des Panels aus Markersequenzen zu erhalten.

## Patentansprüche

1. Verfahren zur Identifizierung und/oder Charakterisierung und/oder Selektion von Markersequenzen mit den Schritten:

    a.) n platzierte Markersequenzen werden auf einem festen Träger mit einer Probe enthaltend hochaffine Binder versetzt und eine Wechselwirkung wird mittels Signalen nachgewiesen,
    b.) mindestens n-1 platzierte Markersequenzen mit Signalintensität aus a.) werden auf einem Träger mit einer gleichen Probe enthaltend hochaffine Binder versetzt und eine Wechselwirkung wird mittels Signalen nachgewiesen,
    c.) optional wird Schritt b.) mit mindestens n-k Markersequenzen wiederholt, wobei $k \geq 1$ gilt,
    d.) n-k ausgewählte Markersequenzen werden auf einen Träger gemeinsam auf einem Locus aufgebracht und mit einer gleichen Probe versetzt und eine Wechselwirkung wird mittels einem Single-Signal nachgewiesen.

2. Verfahren zur Identifizierung und/oder Charakterisierung und/oder Selektion von Markersequenzen nach Anspruch 1, wobei die Markersequenzen aus biologischem Material gewonnen werden, insbesondere Gewebe, native Quellen, Zellen, Bakterien, Viren, Phagen, Prionen, Pflanzen, Tiere, Menschen.

3. Verfahren zur Identifizierung und/oder Charakterisierung und/oder Selektion von Markersequenzen nach Anspruch 1 oder 2, wobei die Markersequenzen mRNA, si-RNA, microRNA, cDNA, Peptid oder Protein, insbesondere Antigene oder Autoantigene, sind oder aus einer Expressionsbibliothek stammen, insbesondere einer mRNA, si-RNA, microRNA, cDNA, Peptid oder Protein-Expressionsbibliothek.

4. Verfahren zur Identifizierung und/oder Charakterisierung und/oder Selektion von Markersequenzen nach einem der vorhergehenden Ansprüche, wobei die hochaffinen Binder Antikörper sind.

5. Verfahren zur Identifizierung und/oder Charakterisierung und/oder Selektion von Markersequenzen nach einem der vorhergehenden Ansprüche, wobei die hochaffinen Binder Autoantikörper sind.

6. Verfahren zur Identifizierung und/oder Charakterisierung und/oder Selektion von Markersequenzen nach einem der vorhergehenden Ansprüche, wobei die Markersequenzen auf einem Träger aufgebracht werden, insbesondere einem Filter, einer Membran, einem magnetischen oder Fluorophor-markiertem Kügelchen, einem Silizium-Wafer, Glas, Metall, Kunststoff, einem Chip, einem massenspektrometrischen Target oder einer Matrix.

7. Verfahren zur Identifizierung und/oder Charakterisierung und/oder Selektion von Markersequenzen nach einem der vorhergehenden Ansprüche, wobei die Probe enthaltend hochaffine Binder, insbesondere Antikörper, Autoantikörper, eine Körperflüssigkeit oder Gewebeauszug ist, insbesondere Blut, Vollblut, Blutplasma, Blutserum, Patientenserum, Urin, Cerebrospinalflüssigkeit, Synovialflüssigkeit.

**8.** Verfahren zur Identifizierung und/oder Charakterisierung und/oder Selektion von Markersequenzen nach einem der vorhergehenden Ansprüche, wobei die Signale zum Nachweis einer Wechselwirkung mit Hilfe von radioaktiven oder fluoreszenzmarkierten Antikörpern erfolgt, insbesondere mittels bioanalytischer Verfahren, wie Western blot (1D und 2D), Immunhistochemie, Antikörperarrays, Luminex, ELISA, Immunfluoreszenz, Radioimmunoassays. massenspektrometrische Verfahren, wie MRM (Multi reaction monitoring) oder AQUA (absolute Quantification.

## Claims

**1.** A method for the identification and/or characterisation and/or selection of marker sequences, comprising the following steps:

a.) n placed marker sequences are mixed on a solid substrate with a sample containing high-affinity binders and an interaction is detected by means of signals,
b.) at least n-1 placed marker sequences having signal intensity from a.) are mixed on a substrate with an identical sample containing high-affinity binders and an interaction is detected by means of signals,
c.) step b.) is optionally repeated with at least n-k marker sequences, wherein $k \geq 1$,
d.) n-k selected marker sequences are applied to a substrate jointly at a locus and are mixed with an identical sample and an interaction is detected by means of a single-signal.

**2.** The method for the identification and/or characterisation and/or selection of marker sequences according to Claim 1, wherein the marker sequences are obtained from biological material, in particular tissue, native sources, cells, bacteria, viruses, phages, prions, plants, animals and humans.

**3.** The method for the identification and/or characterisation and/or selection of marker sequences according to Claim 1 or 2, wherein the marker sequences are mRNA, si-RNA, microRNA, cDNA, peptide or protein, in particular antigens or autoantigens, or originate from an expression library, in particular an mRNA, si-RNA, microRNA, cDNA, peptide or protein expression library.

**4.** The method for the identification and/or characterisation and/or selection of marker sequences according to one of the preceding claims, wherein the high-affinity binders are antibodies.

**5.** The method for the identification and/or characterisation and/or selection of marker sequences according to one of the preceding claims, wherein the high-affinity binders are autoantibodies.

**6.** The method for the identification and/or characterisation and/or selection of marker sequences according to one of the preceding claims, wherein the marker sequences are applied to a substrate, in particular a filter, a membrane, a magnetic or fluorophore labelled bead, a silicon wafer, glass, metal, plastic, a chip, a mass spectrometry target or a matrix.

**7.** The method for the identification and/or characterisation and/or selection of marker sequences according to one of the preceding claims, wherein the sample containing high-affinity binders, in particular antibodies or autoantibodies, is a bodily fluid or tissue extract, in particular blood, whole blood, blood plasma, blood serum, patient serum, urine, cerebrospinal fluid, or synovial fluid.

**8.** The method for the identification and/or characterisation and/or selection of marker sequences according to one of the preceding claims, wherein the signals detect an interaction with the aid of radioactive or fluorescently labelled antibodies, in particular by means of bioanalytical methods, such as Western blot (1D and 2D), immunohistochemistry, antibody arrays, Luminex, ELISA, immunofluorescence, radioimmunoassays or mass spectrometry methods, such as MRM (multi reaction monitoring) or AQUA (absolute quantification).

## Revendications

**1.** Procédé d’identification, et/ou de caractérisation et/ou de sélection de séquences de marqueurs avec les étapes :

a.) n séquences de marqueurs placées sont transposées sur un support solide avec un lieur paraffinique de poids moléculaire élevé contenant un échantillon et une interaction est détectée au moyen de signaux,
b.) au moins n-1 séquences de marqueurs placées avec une intensité de signal provenant de l'étape a.) sont déplacées sur un support avec un même lieur paraffinique de poids moléculaire élevé contenant un même échantillon et une interaction est détectée au moyen de signaux,
c.) l'étape b.) est éventuellement répétée avec au moins n-k séquences de marqueurs, où on a $k \geq 1$,
d.) n-k séquences de marqueurs choisies sont apportées ensemble sur un locus et sont déplacées avec un même échantillon et une interaction est détectée au moyen d'un signal unique.

**2.** Procédé d'identification, et/ou de caractérisation, et/ou de sélection de séquences de marqueurs selon la revendication 1, où les séquences de marqueurs sont récupérées à partir de produits biologiques, notamment de tissus, de sources natives, de cellules, de bactéries, de virus, de phages, de prions, de plantes, d'animaux, d'humains.

**3.** Procédé d'identification, et/ou de caractérisation, et/ou de sélection de séquences de marqueurs selon les revendications 1 ou 2, où les séquences de marqueurs sont l'ARNm, l'ARNsi, le microARN, l'ADNc, un peptide ou une protéine, notamment des antigènes ou des auto-antigènes, où sont issues d'une banque d'expression, notamment d'une banque d'expression d'ARNm, d'ATNsi, de microARN, d'ADNc, de peptides ou de protéines.

**4.** Procédé d'identification, et/ou de caractérisation, et/ou de sélection de séquences de marqueurs selon l'une des revendications précédentes, où les lieurs paraffiniques de poids moléculaire élevé sont des anticorps.

**5.** Procédé d'identification, et/ou de caractérisation, et/ou de sélection de séquences de marqueurs selon l'une des revendications précédentes, où les lieurs paraffiniques de poids moléculaire élevé sont des auto-anticorps.

**6.** Procédé d'identification, et/ou de caractérisation, et/ou de sélection de séquences de marqueurs selon l'une des revendications précédentes, où les séquences de marqueurs sont apportées sur un support, notamment un filtre, une membrane, une bille magnétique ou marquée avec un fluorophore, une plaquette de silicium, du verre, du métal, une matière synthétique, une puce, une cible de spectrométrie de masse ou une matrice.

**7.** Procédé d'identification, et/ou de caractérisation, et/ou de sélection de séquences de marqueurs selon l'une des revendications précédentes, où le lieur parrafinique de poids moléculaire élevé contenant l'échantillon est notamment un anticorps, un auto-anticorps, un liquide corporel ou un extrait de tissu, en particulier, du sang, du sang entier, du plasma sanguin, du sérum sanguin, du sérum de patient, de l'urine, du liquide cérébrospinal, du liquide synovial.

**8.** Procédé d'identification, et/ou de caractérisation, et/ou de sélection de séquences de marqueurs selon l'une des revendications précédentes, où les signaux de détection d'une interaction sont effectués à l'aide d'anticorps marqués radioactifs ou fluorescents, notamment au moyen de procédés bio-analytiques comme le transfert de protéines Western blot (1D et 2D), l'immunohistochimie, les puces à anticorps, l'équipement Luminex, le dosage ELISA, l'immunofluorescence, les dosages radio-immunologiques, les procédés de spectrométrie de masse, comme la technique MRM (suivi multi-réactions) ou la technique AQUA (quantification absolue).

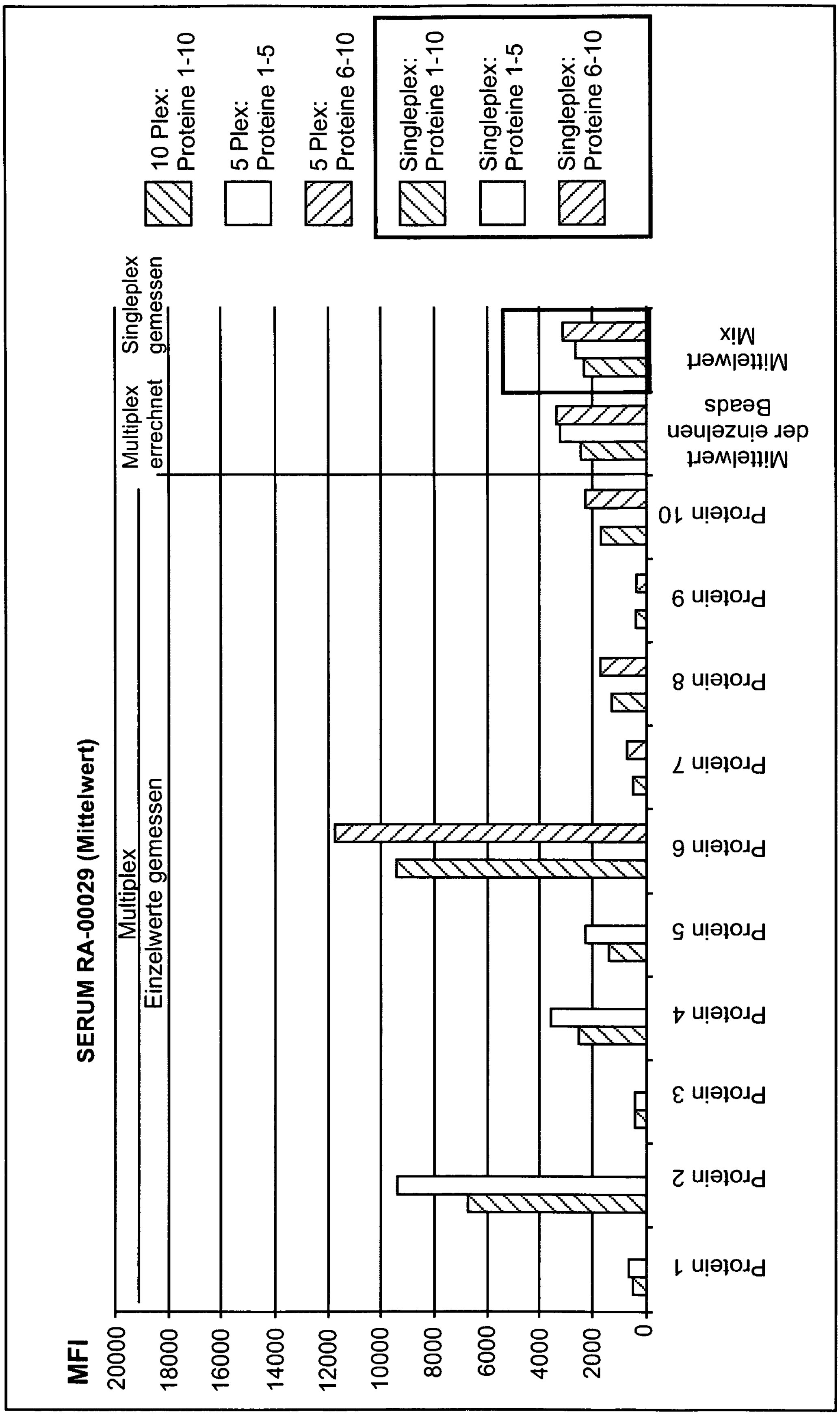
SERUM RA-00029 (Mittelwert)
MFI
20000
18000
16000
14000
12000
10000
8000
6000
4000
2000
0
Multiplex
Einzelwerte gemessen
Multiplex
errechnet
Singleplex
gemessen
Protein 1
Protein 2
Protein 3
Protein 4
Protein 5
Protein 6
Protein 7
Protein 8
Protein 9
Protein 10
Mittelwert
der einzelnen
Beads
Mittelwert
Mix
10 Plex:
Proteine 1-10
5 Plex:
Proteine 1-5
5 Plex:
Proteine 6-10
Singleplex:
Proteine 1-10
Singleplex:
Proteine 1-5
Singleplex:
Proteine 6-10

Fig. 1

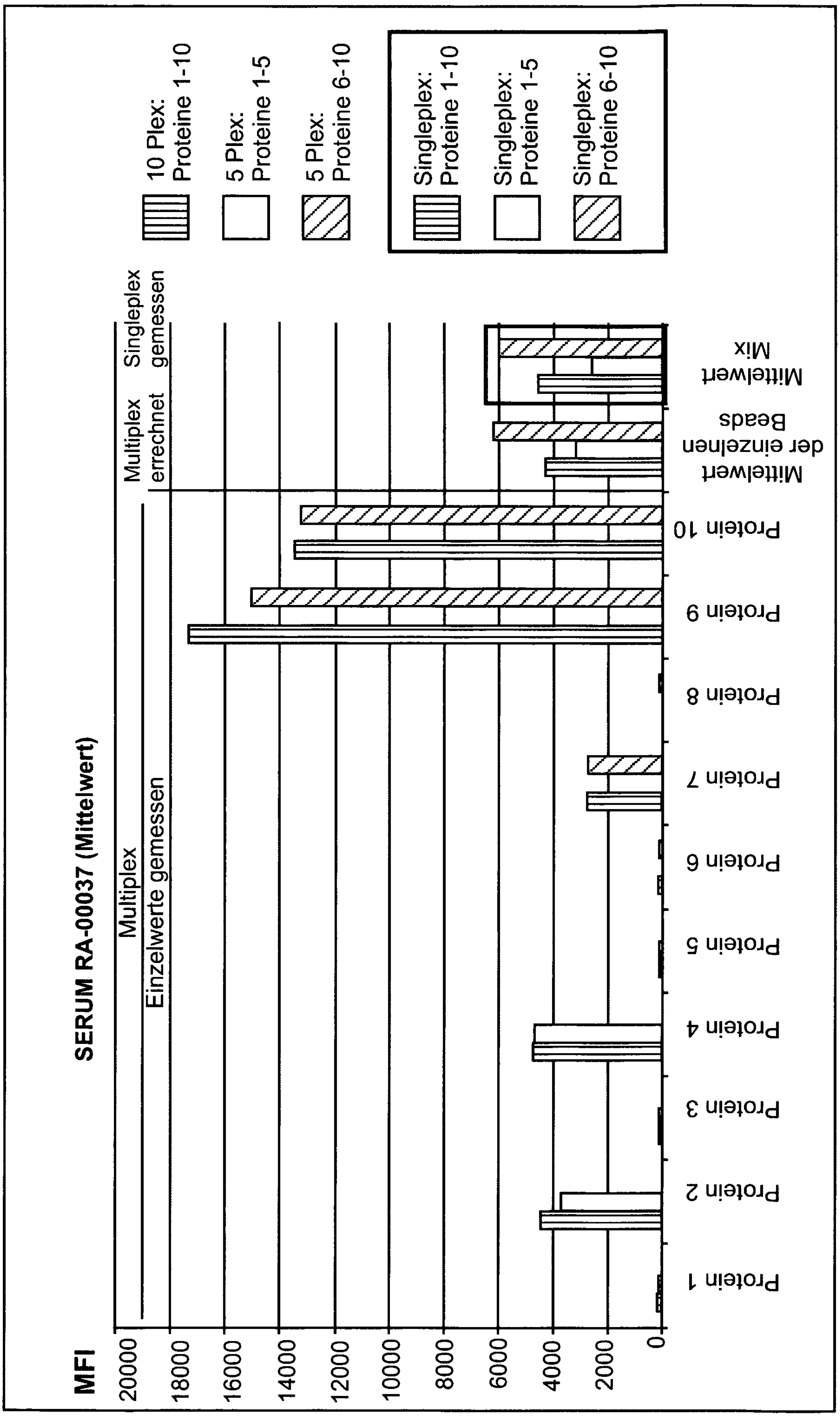
SERUM RA-00037 (Mittelwert)
MFI
20000
18000
16000
14000
12000
10000
8000
6000
4000
2000
0
Multiplex
Einzelwerte gemessen
Multiplex
errechnet
Singleplex
gemessen
Protein 1
Protein 2
Protein 3
Protein 4
Protein 5
Protein 6
Protein 7
Protein 8
Protein 9
Protein 10
Mittelwert der einzelnen Beads
Mittelwert Mix
10 Plex: Proteine 1-10
5 Plex: Proteine 1-5
5 Plex: Proteine 6-10
Singleplex: Proteine 1-10
Singleplex: Proteine 1-5
Singleplex: Proteine 6-10

Fig. 2

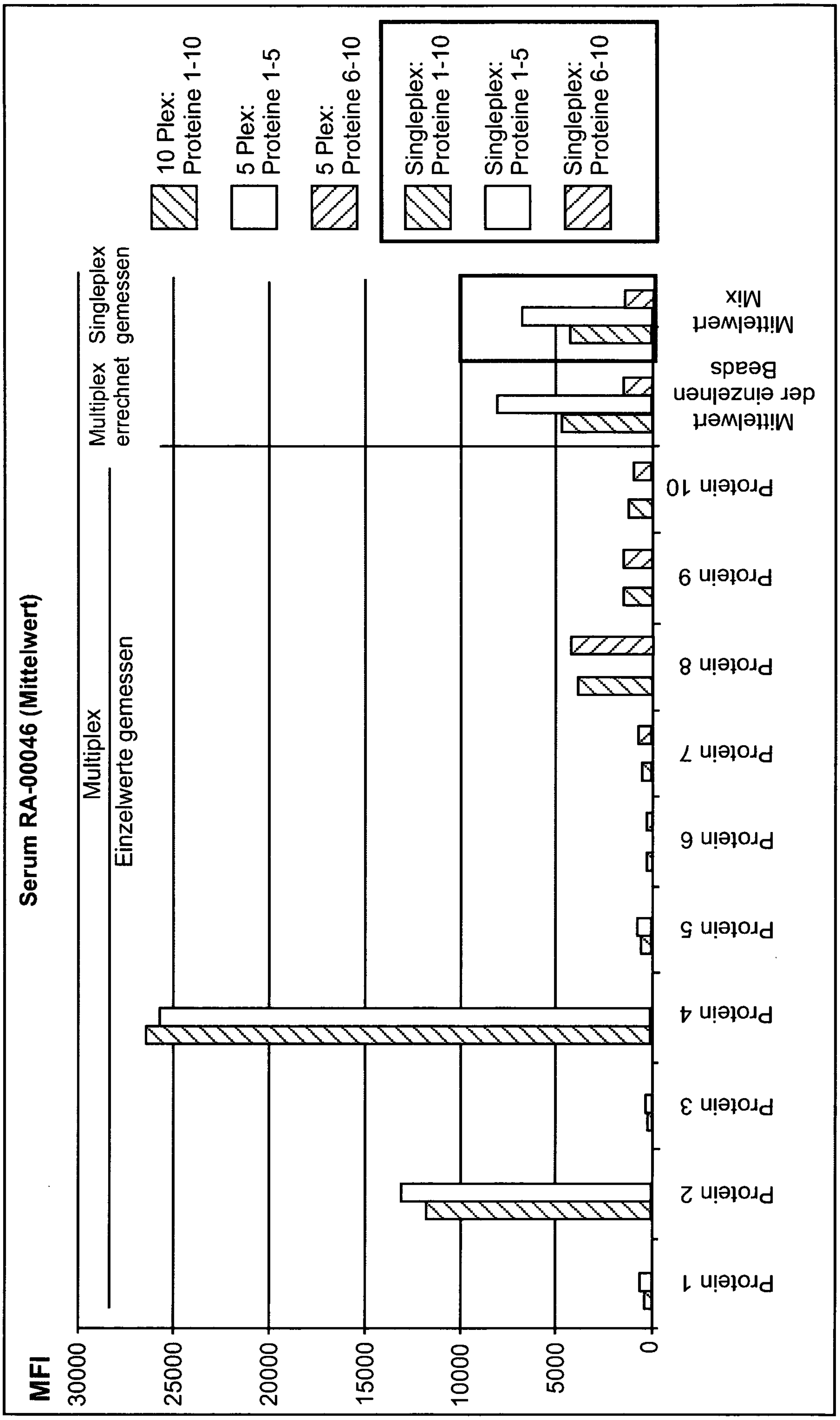
Serum RA-00046 (Mittelwert)
MFI
30000
25000
20000
15000
10000
5000
0
Multiplex
Einzelwerte gemessen
Multiplex
errechnet
Singleplex
gemessen
Protein 1
Protein 2
Protein 3
Protein 4
Protein 5
Protein 6
Protein 7
Protein 8
Protein 9
Protein 10
Mittelwert
der einzelnen
Beads
Mittelwert
Mix
10 Plex:
Proteine 1-10
5 Plex:
Proteine 1-5
5 Plex:
Proteine 6-10
Singleplex:
Proteine 1-10
Singleplex:
Proteine 1-5
Singleplex:
Proteine 6-10

Fig. 3

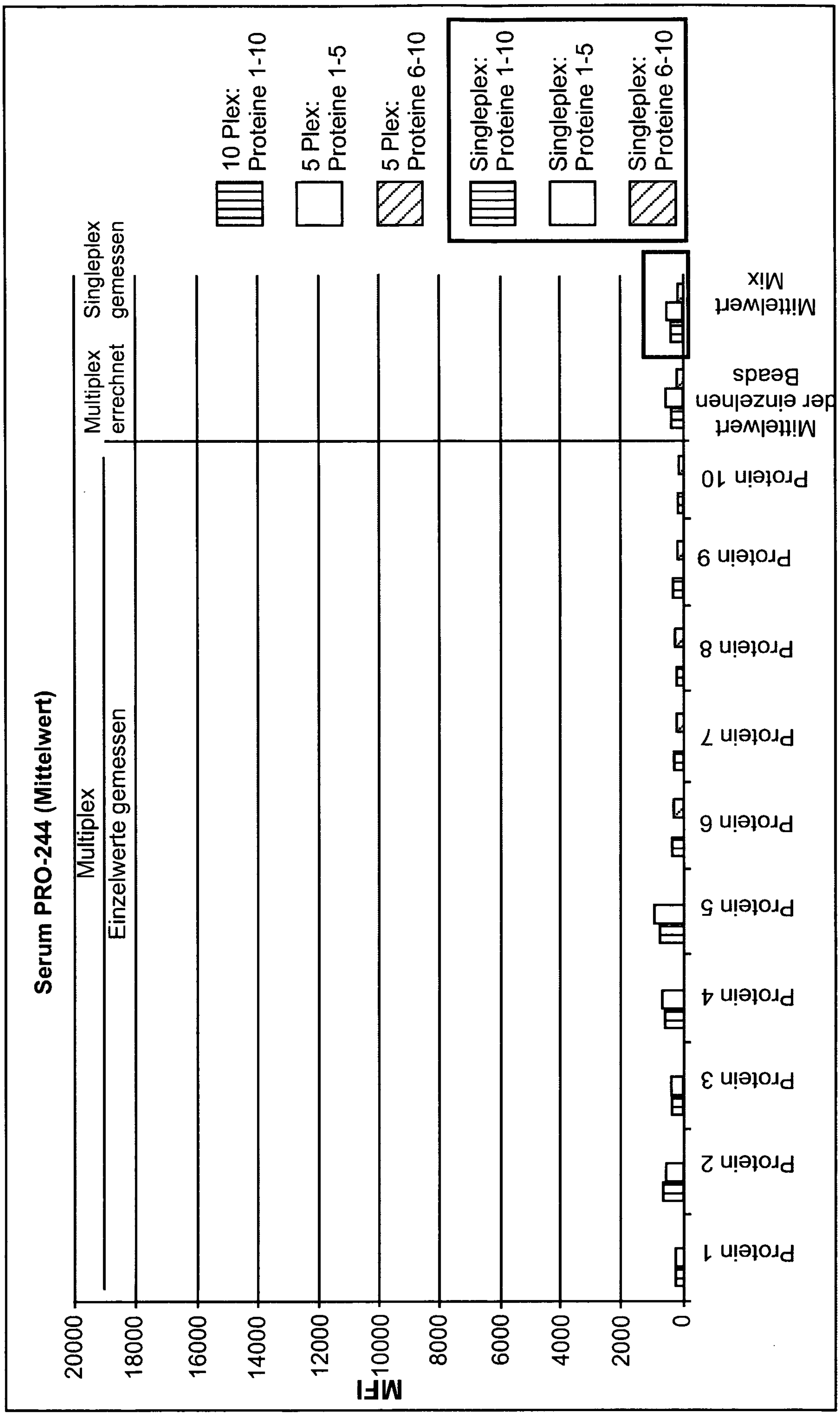
Serum PRO-244 (Mittelwert)
MFI
20000
18000
16000
14000
12000
10000
8000
6000
4000
2000
0
Multiplex
Einzelwerte gemessen
Multiplex
errechnet
Singleplex
gemessen
10 Plex:
Proteine 1-10
5 Plex:
Proteine 1-5
5 Plex:
Proteine 6-10
Singleplex:
Proteine 1-10
Singleplex:
Proteine 1-5
Singleplex:
Proteine 6-10
Protein 1
Protein 2
Protein 3
Protein 4
Protein 5
Protein 6
Protein 7
Protein 8
Protein 9
Protein 10
Mittelwert
der einzelnen
Beads
Mittelwert
Mix

Fig. 4

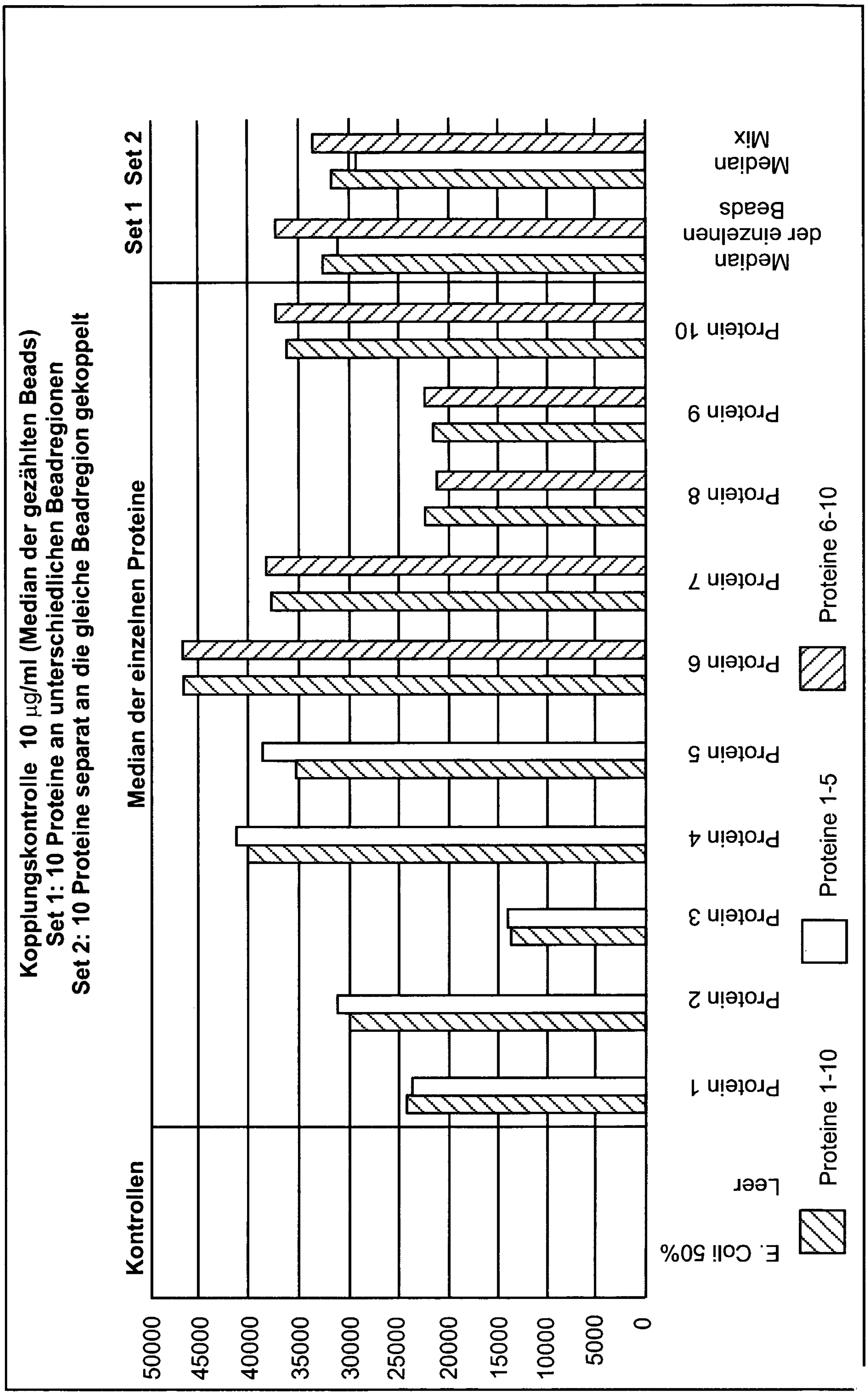
Kopplungskontrolle 10 µg/ml (Median der gezählten Beads)
Set 1: 10 Proteine an unterschiedlichen Beadregionen
Set 2: 10 Proteine separat an die gleiche Beadregion gekoppelt
Kontrollen
Median der einzelnen Proteine
Set 1
Set 2
50000
45000
40000
35000
30000
25000
20000
15000
10000
5000
0
E. Coli 50%
Leer
Protein 1
Protein 2
Protein 3
Protein 4
Protein 5
Protein 6
Protein 7
Protein 8
Protein 9
Protein 10
Median der einzelnen Beads
Median Mix
Proteine 1-10
Proteine 1-5
Proteine 6-10

Fig. 5

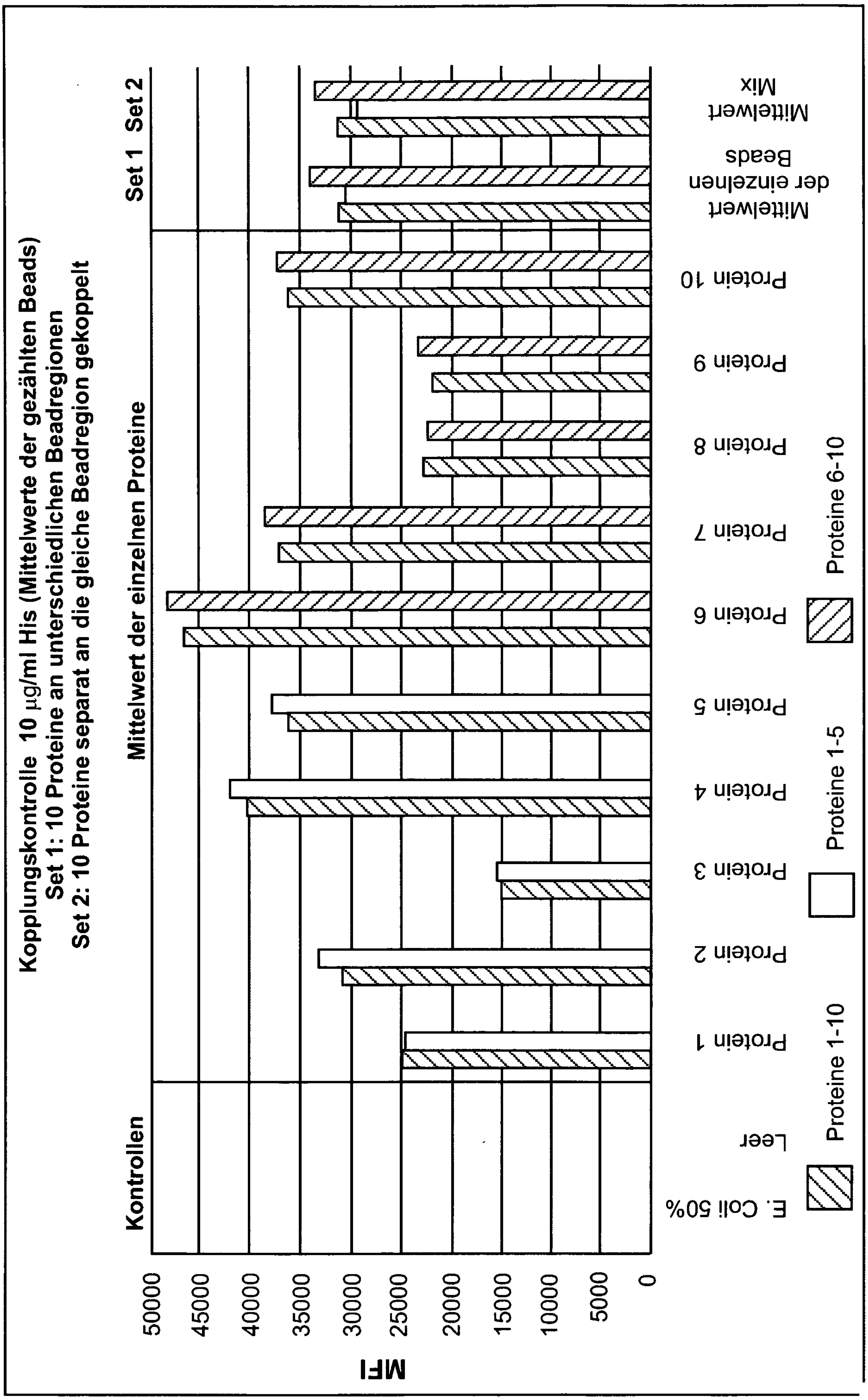

Kopplungskontrolle 10 µg/ml His (Mittelwerte der gezählten Beads)
Set 1: 10 Proteine an unterschiedlichen Beadregionen
Set 2: 10 Proteine separat an die gleiche Beadregion gekoppelt
Kontrollen
Mittelwert der einzelnen Proteine
Set 1
Set 2
MFI
50000
45000
40000
35000
30000
25000
20000
15000
10000
5000
0
E. Coli 50%
Leer
Protein 1
Protein 2
Protein 3
Protein 4
Protein 5
Protein 6
Protein 7
Protein 8
Protein 9
Protein 10
Mittelwert der einzelnen Beads
Mittelwert Mix
Proteine 1-10
Proteine 1-5
Proteine 6-10


Fig. 6

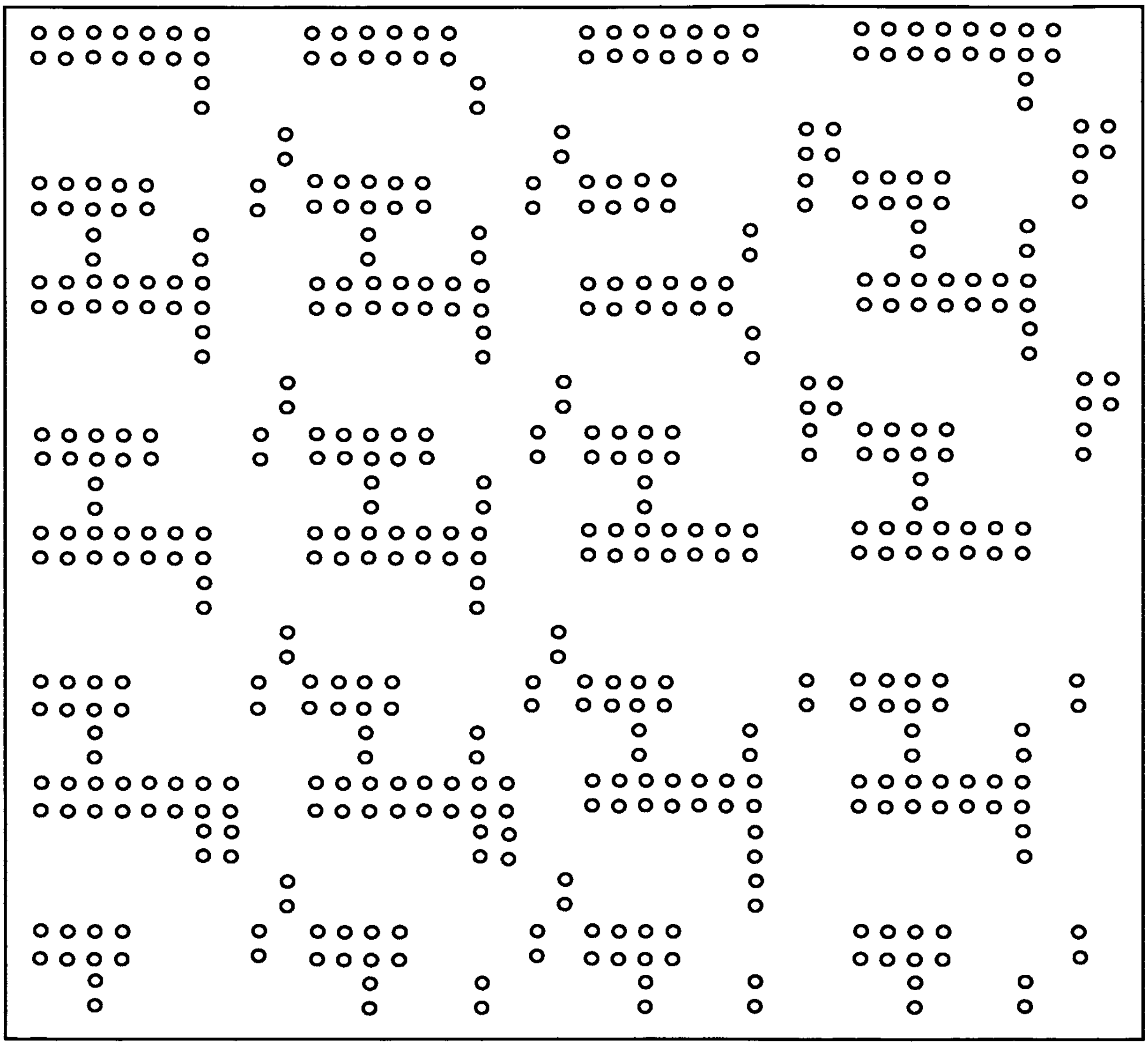

Fig. 7

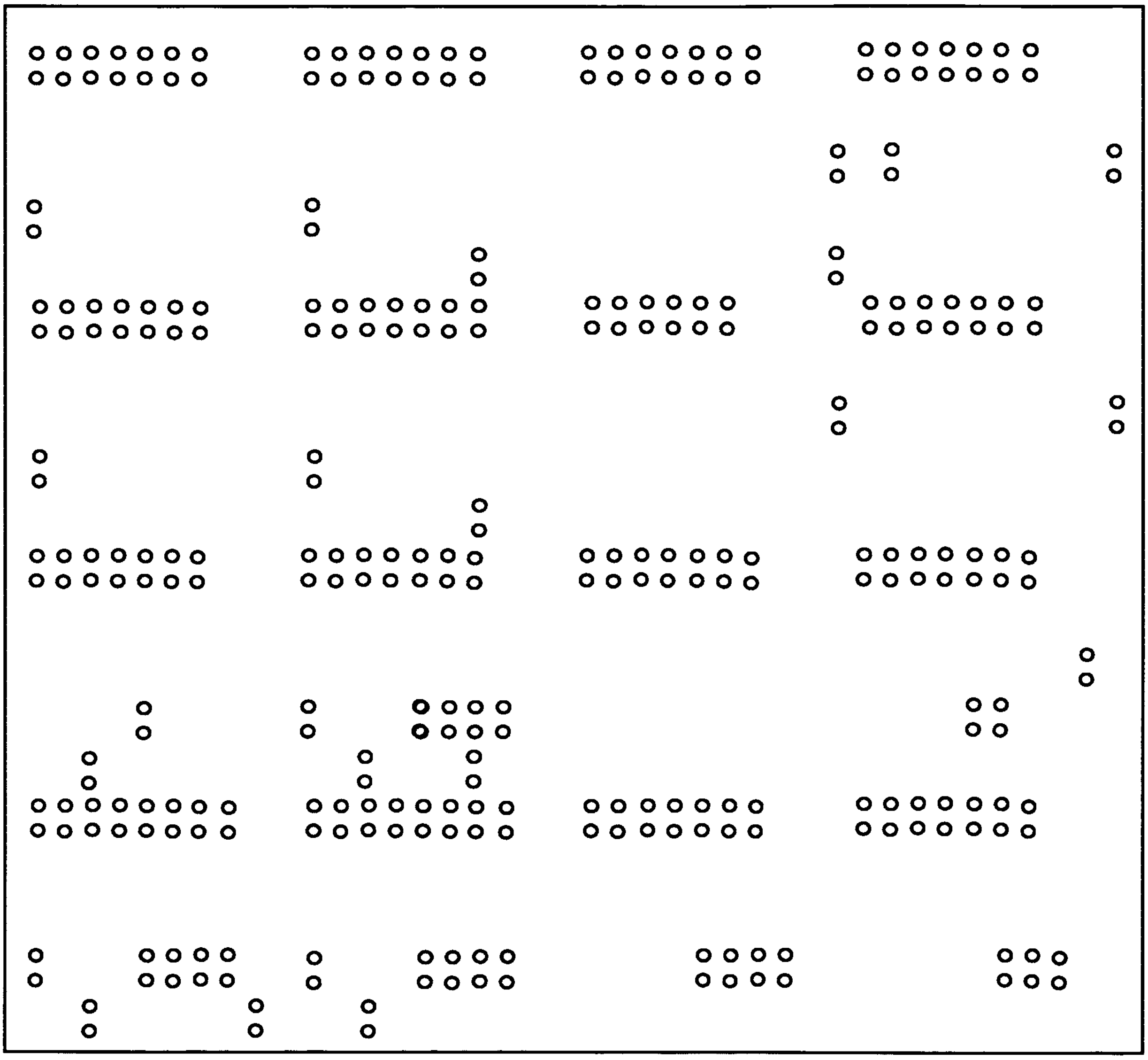

Fig. 8

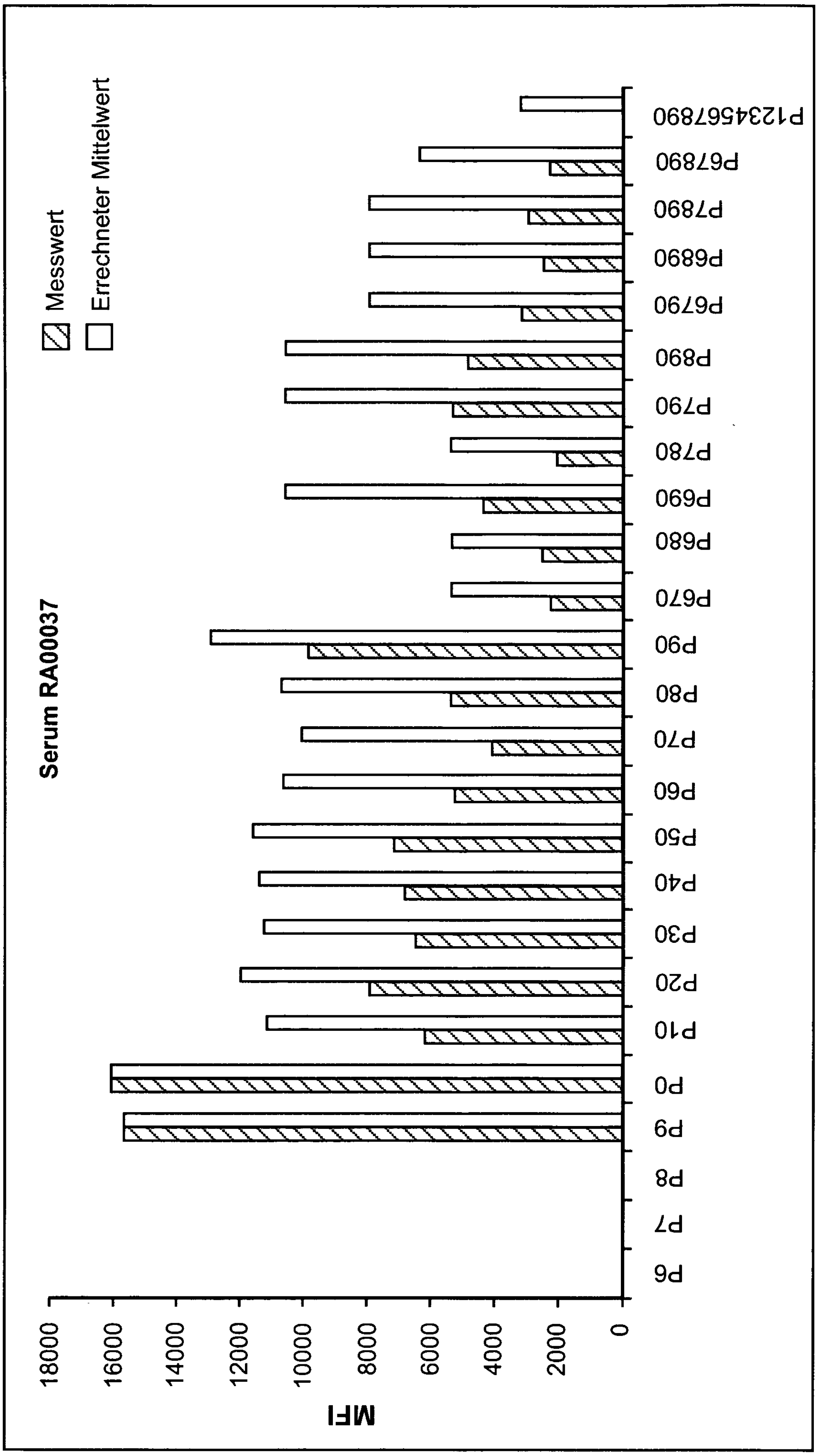
Serum RA00037
Messwert
Errechneter Mittelwert
MFI
18000
16000
14000
12000
10000
8000
6000
4000
2000
0
P6
P7
P8
P9
P0
P10
P20
P30
P40
P50
P60
P70
P80
P90
P670
P680
P690
P780
P790
P890
P6790
P6890
P7890
P67890
P1234567890

Fig. 9

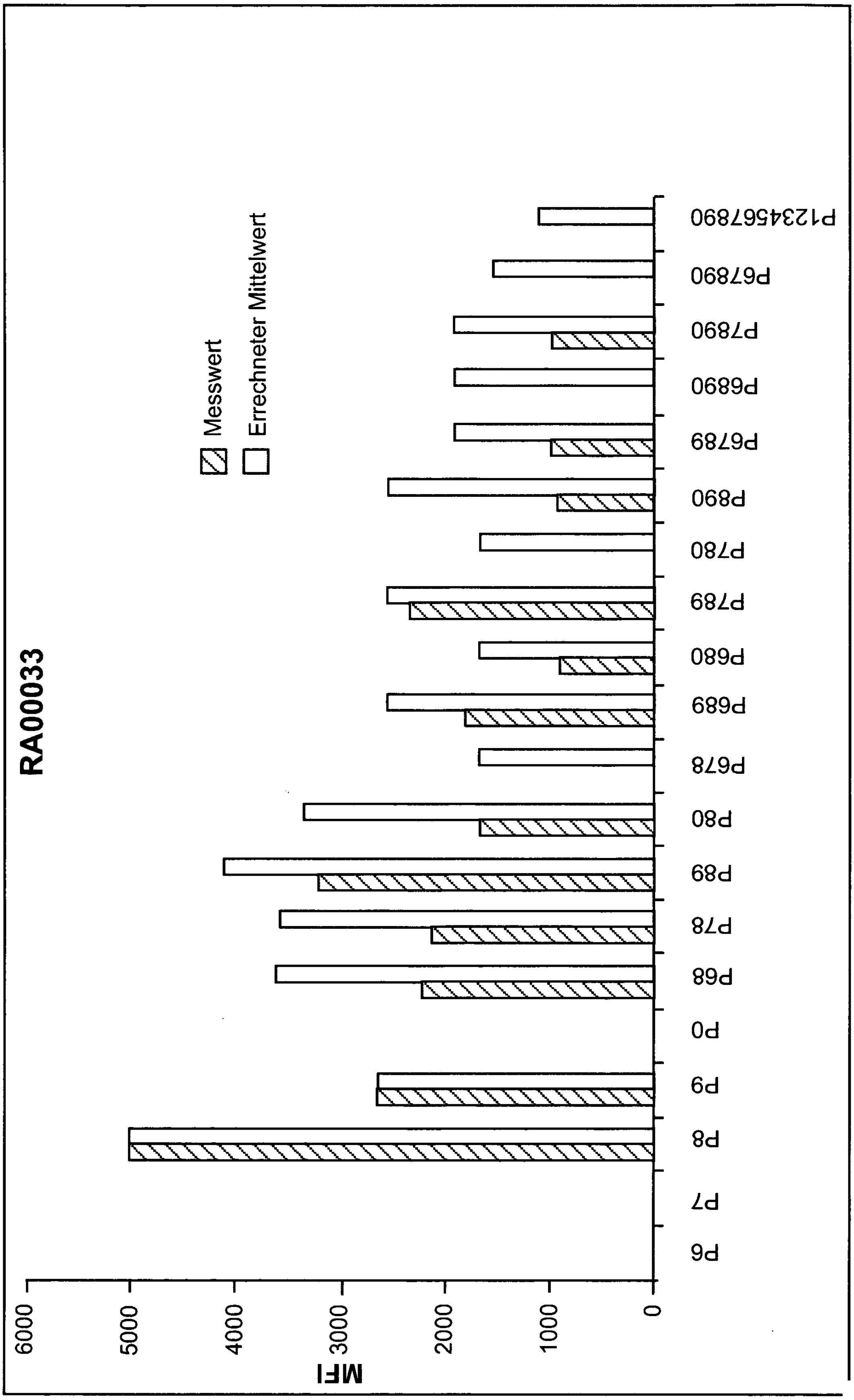
RA00033
Messwert
Errechneter Mittelwert
MFI
6000
5000
4000
3000
2000
1000
0
P6
P7
P8
P9
P0
P68
P78
P89
P80
P678
P689
P680
P789
P780
P890
P6789
P6890
P7890
P67890
P1234567890

Fig. 10

| | RA00026 | RA00027 | RA00028 | RA00029 | RA00030 | RA00031 | RA00032 | RA00033 | RA00035 | RA00036 | RA00037 | RA00038 | RA00039 | RA00040 | RA00041 | RA00042 | RA00043 | RA00044 | RA00045 | RA00046 |
|---|---|---|---|---|---|---|---|---|---|---|---|---|---|---|---|---|---|---|---|---|
| P1 | 0 | 0 | 0 | 0 | 0 | 0 | 1062 | 1367 | 2592 | 0 | 0 | 0 | 1216 | 0 | 0 | 0 | 0 | 0 | 0 | 0 |
| P2 | 0 | 5438 | 0 | 0 | 0 | 0 | 0 | 0 | 0 | 0 | 0 | 0 | 0 | 0 | 0 | 0 | 0 | 0 | 2295 | 0 |
| P3 | 883 | 0 | 0 | 0 | 0 | 223 | 573 | 1573 | 985 | 3208 | 0 | 0 | 1927 | 0 | 0 | 0 | 0 | 0 | 0 | 0 |
| P4 | 2817 | 0 | 0 | 0 | 0 | 0 | 4606 | 492 | 0 | 1209 | 0 | 0 | 271 | 5017 | 0 | 0 | 0 | 0 | 0 | 7955 |
| P5 | 0 | 0 | 0 | 0 | 0 | 0 | 0 | 0 | 0 | 0 | 0 | 0 | 0 | 0 | 0 | 0 | 6128 | 0 | 0 | 0 |
| P6 | 0 | 0 | 0 | 0 | 0 | 0 | 0 | 0 | 0 | 0 | 0 | 0 | 0 | 0 | 0 | 0 | 0 | 0 | 0 | 0 |
| P7 | 2519 | 0 | 0 | 1921 | 3247 | 2405 | 254 | 0 | 0 | 0 | 0 | 0 | 710 | 0 | 0 | 0 | 0 | 0 | 0 | 0 |
| P8 | 0 | 0 | 0 | 0 | 0 | 0 | 0 | 5010 | 0 | 0 | 0 | 0 | 0 | 0 | 0 | 0 | 0 | 0 | 0 | 0 |
| P9 | 0 | 0 | 0 | 0 | 0 | 0 | 0 | 2631 | 0 | 0 | 15643 | 0 | 1692 | 0 | 0 | 0 | 0 | 0 | 0 | 0 |
| P0 | 0 | 0 | 5230 | 0 | 0 | 0 | 2139 | 0 | 0 | 0 | 16034 | 0 | 0 | 0 | 3984 | 0 | 0 | 0 | 0 | 0 |

**MFI**

- (empty box) < 1000
- (box with single diagonal) 1000 - 10000
- (box with crossed diagonals) > 10000

Fig. 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9957311 A
- WO 9957312 A
- WO 2011038138 A1
- US 2005019843 A1
- WO 2010000874 A


### In der Beschreibung aufgeführte Nicht-Patentliteratur


- **HEYMAN, J.A. ; CORNTHWAITE, J. ; FONCERRADA, L. ; GILMORE, J.R. ; GONTANG, E. ; HARTMAN, K.J. ; HERNANDEZ, C.L. ; HOOD, R. ; HULL, H.M. ; LEE, W.Y.** Genome-scale cloning and expression of individual open reading frames using topoisomerase I-mediated ligation. *Genome Res,* 1999, vol. 9, 383-392
- **KERSTEN, B. ; FEILNER, T. ; KRAMER, A. ; WEHRMEYER, S. ; POSSLING, A. ; WITT, I. ; ZANOR, M.I. ; STRACKE, R. ; LUEKING, A. ; KREUTZBERGER, J.** Generation of Arabidopsis protein chip for antibody and serum screening. *Plant Molecular Biology,* 2003, vol. 52, 999-1010
- **REBOUL, J. ; VAGLIO, P. ; RUAL, J.F. ; LAMESCH, P. ; MARTINEZ, M. ; ARMSTRONG, C.M. ; LI, S. ; JACOTOT, L. ; BERTIN, N. ; JANKY, R.** C. elegans ORFeome version 1.1: experimental verification of the genome annotation and resource for proteome-scale protein expression. *Nat Genet,* 2003, vol. 34, 35-41
- **WALHOUT, A.J. ; TEMPLE, G.F. ; BRASCH, M.A. ; HARTLEY, J.L. ; LORSON, M.A. ; VAN DEN HEUVEL, S. ; VIDAL, M.** GATEWAY recombinational cloning: application to the cloning of large numbers of open reading frames or ORFeomes. *Methods Enzymol,* 2000, vol. 328, 575-592
- **BÜSSOW, K. ; CAHILL, D. ; NIETFELD, W. ; BANCROFT, D. ; SCHERZINGER, E. ; LEHRACH, H. ; WALTER, G.** A method for global protein expression and antibody screening on high-density filters of an arrayed cDNA library. *Nucleic Acids Research,* 1998, vol. 26, 5007-5008
- **BÜSSOW, K. ; NORDHOFF, E. ; LÜBBERT, C. ; LEHRACH, H. ; WALTER, G.** A human cDNA library for high-throughput protein expression screening. *Genomics,* 2000, vol. 65, 1-8
- **HOLZ, C. ; LUEKING, A. ; BOVEKAMP, L. ; GUTJAHR, C. ; BOLOTINA, N. ; LEHRACH, H. ; CAHILL, D.J.** A human cDNA expression library in yeast enriched for open reading frames. *Genome Res,* 2001, vol. 11, 1730-1735
- **LUEKING, A. ; HOLZ, C. ; GOTTHOLD, C. ; LEHRACH, H. ; CAHILL, D.** A system for dual protein expression in Pichia pastoris and Escherichia coli. *Protein Expr. Purif.,* 2000, vol. 20, 372-378
- **BRAUN P. ; HU, Y. ; SHEN, B. ; HALLECK, A. ; KOUNDINYA, M. ; HARLOW, E. ; LABAER, J.** Proteome-scale purification of human proteins from bacteria. *Proc Natl Acad Sci U S A,* 2002, vol. 99, 2654-2659
- **LUEKING, A. ; HORN, M. ; EICKHOFF, H. ; BÜSSOW, K. ; LEHRACH, H. ; WALTER, G.** Protein microarrays for gene expression and antibody screening. *Analytical Biochemistry,* 1999, vol. 270, 103-111
- **LAL et al.** Antibody arrays: An embryonic but rapidly growing technology. *DDT,* 2002, vol. 7, 143-149
- **KUSNEZOW et al.** Antibody microarrays: An evaluation of production parameters. *Proteomics,* 2003, vol. 3, 254-264
- Screening autoantibody profiles in systemic rheumatic disease with a diagnostic protein microarray that uses a filtration-assisted nanodot array luminometric immunoassay (NALIA). **MCBRIDE JEFFREY D et al.** CLINICAL CHEMISTRY. AMERICAN ASSOCIATION FOR CLINICAL CHEMISTRY, 01. Mai 2008, vol. 54, 883-890
- **J. SAMBROOK ; E.F. FRITSCH ; T. MANIATIS.** Molecular cloning: A laboratory manual. Cold Spring Habor Laboratory Press, 1989
- **AUSUBEL.** Current Protocols in Molecular Biology. Green Publishing Associates and Wiley Interscience, 1989
- **TERPE et al.** *Terpe T Appl Microbiol Biotechnol.,* Januar 2003, vol. 60 (5), 523-33
- **SAMBROOK et al.** Molecular Cloning, A laboratory handbook. CSH press, 1989
- **ROSALYN S. YALOW ; SOLOMON A. BERSON.** Immunoassays of endogenous plasma insulin in man. *From the Radioisotope Service, Veterans Administration Hospital,* 07. Marz 1960
- *J. Clin. Invest.,* vol. 39, 1157-75

- Enzym-linked immunosorbent assay (ELISA) Quantitative assay of immunoglobulin G. Immunochemistry. Pergamon Press, 1971, vol. 8, 871-874